(19) Europäisches Patentamt
European Patent Office
Office européen des brevets

(11) EP 3 848 377 A1

(12) EUROPEAN PATENT APPLICATION
published in accordance with Art. 153(4) EPC

(43) Date of publication:
**14.07.2021 Bulletin 2021/28**

(21) Application number: **19867204.0**

(22) Date of filing: **26.09.2019**

(51) Int Cl.:
*C07D 487/14* (2006.01) *C07D 471/14* (2006.01)
*C07D 519/00* (2006.01) *A61K 31/4375* (2006.01)
*A61K 31/437* (2006.01) *A61P 35/00* (2006.01)

(86) International application number:
**PCT/CN2019/108298**

(87) International publication number:
**WO 2020/063788 (02.04.2020 Gazette 2020/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **27.09.2018 PCT/CN2018/107902**

(71) Applicant: **Betta Pharmaceuticals Co., Ltd**
**Yuhang**
**Hangzhou**
**Zhejiang 311100 (CN)**

(72) Inventors:
- **GAO, Jinheng**
  **Beijing 100176 (CN)**
- **SUN, Zhongxin**
  **Beijing 100176 (CN)**
- **ZHANG, Yun**
  **Beijing 100176 (CN)**
- **XU, Xiaofeng**
  **Beijing 100176 (CN)**
- **LIU, Xiangyong**
  **Beijing 100176 (CN)**
- **WANG, Jiabing**
  **Beijing 100176 (CN)**
- **DING, Lieming**
  **Hangzhou, Zhejiang 311100 (CN)**

(74) Representative: **Icosa**
**83 avenue Denfert-Rochereau**
**75014 Paris (FR)**

# (54) FGFR4 INHIBITOR AND USE THEREOF

(57) Disclosed is a compound as a fibroblast growth factor receptor 4 (FGFR4) inhibitor (as shown in formula (I)), and a pharmaceutical composition thereof and a preparation method therefor, as well as the use of same in the treatment of FGFR4-mediated diseases. The above-mentioned compounds act by participating in a number of processes, such as regulating cell proliferation, apoptosis, migration, neovascularization.

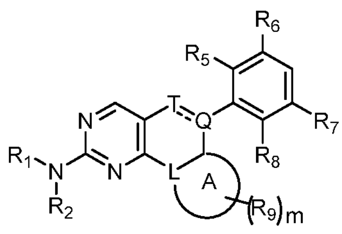

Formula (I)

EP 3 848 377 A1

## Description

### FIELD OF THE INVENTION

The present invention relates to a series of compound which act as inhibitors of fibroblast growth factor receptors 4 (FGFR4), as well as the preparation method and pharmaceutical composition thereof. The present invention further relates to a use of the above compounds or pharmaceutical composition thereof in the treatment of FGFR4-mediated disorders.

### BACKGROUND OF THE INVENTION

Protein kinases are enzymes that catalyze the phosphorylation of proteins, in most instances, this phosphorylation occurs on the residues of the serine (ser), threonine (thr) and tyrosine (tyr) of the protein. Many aspects of cell life processes (eg. cell growth, differentiation, proliferation, cell cycle and survival) are dependent on the activity of protein kinase. Furthermore, many diseases (eg. cancer and inflammation) are associated with the abnormal activity of protein kinase.

It has been found that Protein Tyrosine Kinase, PTK have more than 100 family members so far, which play an important role in regulating cell differentiation, growth and proliferation. According to the structure of the PTK, it can be divided into two types: receptor PTK which is also called as transmembrane PTK and non-receptor PTK which is also called as intracellular PTK.

Fibroblast growth factor receptors (FGFR) is a member of receptor tyrosine kinase (RTK) superfamily, which regulates the cell proliferation, differentiation and migration in different tissues through the complex signal transmission pathways by combining with fibroblast growth factor (FGF) (Jouanneau J et al. Oncogene, 1999, 18:327-333). FGFR is a single-chain glycoprotein consisted of an extracellular region, a single transmembrane region and a tyrosine kinase region in the cytoplasm. The extracellular region is composed of a leader peptide and three immunoglobulin domains. The FGFRs family comprises FGFR1, FGFR2, FGFR3 and FGFR4. The FGFR4 gene is located at 5q35.1 of chromosome, which is about 11.3 kb in length with 18 exons (Kostrzewa M. Mammalian genome, 1998, 9(2): 131-135). FGFR4 protein is an important member of the FGFR tyrosine kinase family, its' 388th amino acid is located in the highly conserved transmembrane region of RTK structure, the changes in the pathophysiological function of FGFR4 protein caused by the changes of this structural can enhance the activity of tyrosine kinase. FGFR4 protein is a type of transmembrane tyrosine kinase receptor with autophosphorylation activity, which plays an important role in embryonic development, tissue repair and angiogenesis. (Eswarakumar V P et al. Cytokine Growth Factor Rev, 2005, 16(2): 139-149).

FGFR4 signaling pathway: Mediated by heparin or heparinoid, the ligand binds to FGFR4, causing the FGFR4 monomer to dimerize, with the tyrosine phosphorylation of the C-terminus of the cytoplasm, the kinase insertion region and the proximal membrane region, FGFR4 is activated by the phosphorylation of the kinase domain of A loop (activation loop). (Schlessinger J et al. Mol Cell, 2000, 6:743-750). The activated FGFR4 mainly has two intracellular agents which are Phospholipase C and FGF receptor substrate 2 (FRS2) (Dailey L et al. Cytokine Growth Factor Rev, 2005, 16:233-247).

When FGFR4 is activated, the Src homology region 2 (SH2) domain of phospholipase C binds to its activated C-terminal tyrosine, causing the phosphorylateion of the PLC and binds to the C-terminal tyrosine site. The activated PLC hydrolyzes its substrate 4,5-diphosphate phosphatidylinositol (PIP2) to form diacylglycerol (DAG) and inositol triphosphate (IP3). IP3 binds to specific receptors in the cell to stimulate the intracellular calcium pool to release $Ca^{2+}$, $Ca^{2+}$ binds to calmodulin to activate $Ca^{2+}$/calmodulin-dependent protein kinase.Besides, both $Ca^{2+}$ and diacylglycerol can activate members of the protein kinase C family. In addition to activating transcription factors, the secondary signal generated by PIP2 hydrolysis can also activate a variety of intracellular reactions.

SOS protein binds to the Src homology region 3 (SH3) domain of growth factor receptor bound 2 (Grb2) to form a Grb2/SOS complex, which can binds to FGFR4 or FGFR substrate 2$\alpha$ (FRS2$\alpha$), wherein FRS2$\alpha$ is connected with phosphotyrosine binding domain (PTB), promoting the exchange of guanosine on Ras to make Ras become Ras-GTP to starting the downstream MAPK signaling pathway.

The autophosphorylation of FGFR4 activates JAK family factors (JAK), the activated JAK cause the phosphorylation of specific signal protein adsorption site on FGFR4, this site can be the docking sites of signal transducer and activator of transcription (STAT) and other signaling molecules.The C-terminal tyrosine residue of the STAT protein is phosphorylated by JAK when STAT protein is absorbed by FGFR4 docking site, and the phosphorylated STAT protein separates from the receptor to form a stable homodimer or heterodimer and then is transferred to the nucleus to interact with gamma interferon activation site (GAS) enhancer family members to activate the transcription of target genes.

Small molecules FGFR4 inhibitors inhibit the proliferation signal mediated by FGFR4 by blocking the combination of the extracellular ligand molecules with receptors or the transmission of intracellular kinase signals. There are many types of FGFR4inhibitors currently under development, the FGFR4 selective inhibitor AZ709 developed by AstraZeneca

shows a good inhibitory effect on cells expressing FGF19 or FGFR4 at high level in vitro experiments, but there's no obvious effect in the in vivo experiments. The FGFR4 selective inhibitor FGF401 developed by Novartis can target FGFR4 specifically to treat the malignant tumors such as liver cancer caused by the overexpression of the FGFR4. The FGFR4 specific inhibitor H3B6527 developed by H3 Biomedicine has a strong anti-tumor activity on FGF19 gene-amplified cells, and isn't found any bile acid-related adverse reactions in the mouse and monkey animal models. Blueprint Medicine has developed and reported a FGFR4-specific inhibitor BLU554 to treat liver cancer and cholangiocarcinoma with overexpression of FGFR4.

With the in-depth study of the structure and the function of FGFR4 and the interaction with other genes, the FGFR4 inhibitors with good specificity and therapeutic effect and low adverse reactions will be developed, and the use of FGFR4 molecular targeted therapy for tumors will be very meaningful.

## SUMMARY OF THE INVENTION

The present invention relates to compounds as an inhibitor of fibroblast growth factor receptors 4 (FGFR4), or pharmaceutical acceptable salts, solvates, chelates, non-covalent complexes or prodrugs thereof. The compounds of the present invention have a general structure as Formula (I):

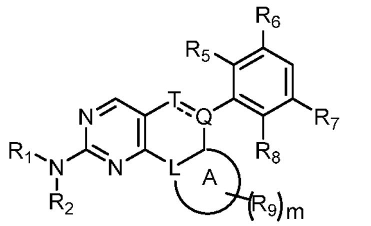

Formula I

wherein,

--- is a single bond or a double bond;

L, Q or T is each independently selected from the group consisting of O, N, C, CH, $CH_2$ and $CR_{17}$;

ring A is $C_{6-10}$aryl, substituted $C_{6-10}$aryl, $C_{5-10}$heteroaryl, substituted $C_{5-10}$heteroaryl, $C_{5-10}$heterocyclyl, substituted $C_{5-10}$heterocyclyl; wherein the $C_{5-10}$heteroaryl or $C_{5-10}$heterocyclyl optionally containing 1, 2 or 3 heteroatoms independently selected from N, O and S;

Ri is selected from the group consisting of hydrogen, halogen, $C_{1-8}$alkyl, substituted $C_{1-8}$alkyl, Ci-salkoxy, substituted $C_{1-8}$alkoxy, $C_{2-8}$alkenyl, substituted $C_{2-8}$alkenyl, $C_{2-8}$alkynyl and substituted $C_{2-8}$alkynyl;

$R_2$ is selected from the group consisting of $C_{3-10}$cycloalkyl, substituted $C_{3-10}$cycloalkyl, $C_{3-10}$heterocyclyl, substituted $C_{3-10}$heterocyclyl, $C_{6-10}$aryl, substituted $C_{6-10}$aryl, $C_{6-10}$heteroaryl and substituted $C_{6-10}$heteroaryl, wherein the $C_{3-10}$heterocyclyl or $C_{6-10}$heteroaryl optionally containing 1 or 2 heteroatoms selected from N and O ;

$R_2$ is optionally substituted with 1-2 $R_{13}$ substituents;

$R_{13}$ is selected from the group consisting of hydroxyl, halogen, $C_{1-8}$alkyl, substituted $C_{1-8}$alkyl, $C_{2-8}$alkenyl, substituted $C_{2-8}$alkenyl, $C_{2-8}$alkynyl, substituted $C_{2-8}$alkynyl, $C_{1-8}$alkoxy, substituted $C_{1-8}$alkoxy, $C_{6-10}$aryl, substituted $C_{6-10}$aryl, $C_{3-10}$cycloalkyl, substituted $C_{3-10}$cycloalkyl, $C_{3-10}$heterocyclyl, substituted $C_{3-10}$heterocyclyl, $C_{5-10}$heteroaryl, substituted $C_{5-10}$heteroaryl, $-NR_{14}R_{15}$ and $-CO-R_{16}$, wherein $C_{3-10}$heterocyclyl or $C_{5-10}$heteroaryl optionally containing 1, 2 or 3 heteroatoms selected from N and O;

$R_{13}$ is optionally substituted with 0-1 $R_{18}$ substituent;

$R_{18}$ is selected from the group consisting of hydroxyl, halogen, $C_{1-8}$alkyl, substituted $C_{1-8}$alkyl, $C_{2-8}$alkenyl, substituted $C_{2-8}$alkenyl, $C_{2-8}$alkynyl, substituted $C_{2-8}$alkynyl, $C_{1-8}$alkoxy, substituted $C_{1-8}$alkoxy, $C_{6-10}$aryl, substituted $C_{6-10}$aryl, $C_{3-10}$cycloalkyl, substituted $C_{3-10}$cycloalkyl, $C_{3-10}$heterocyclyl, substituted $C_{3-10}$heterocyclyl, $C_{5-10}$heteroaryl and substituted $C_{5-10}$heteroaryl, wherein $C_{3-10}$heterocyclyl or $C_{5-10}$heteroaryl containing 1, 2 or 3 heteroatoms selected from N and O;

$R_5$, $R_6$, $R_7$ and $R_8$ is independently selected from the group consisting of hydroxyl, halogen, Ci-salkoxy, substituted Ci-salkoxy, Ci-salkyl, substituted $C_{1-8}$alkyl, $C_{2-8}$alkenyl, substituted $C_{2-8}$alkenyl, $C_{2-8}$alkynyl, substituted $C_{2-8}$alkynyl, $C_{3-8}$cycloalkyl, substituted $C_{3-8}$cycloalkyl, $C_{6-10}$aryl, substituted $C_{6-10}$aryl, $C_{5-10}$heteroaryl, substituted $C_{5-10}$heteroaryl, $C_{3-10}$heterocyclyl and substituted $C_{3-10}$ heterocyclyl;

$R_9$ is selected from the group consisting of H, halogen, amino, cyano, Ci-salkyl, substituted $C_{1-8}$alkyl, Ci-salkoxy, substituted Ci-salkoxy, $C_{2-8}$alkenyl, substituted $C_{2-8}$alkenyl, $C_{2-8}$alkynyl, substituted $C_{2-8}$alkynyl, $C_{6-10}$aryl, substi-

tuted $C_{6-10}$aryl, $C_{3-8}$cycloalkyl, substituted $C_{3-8}$cycloalkyl, $C_{3-10}$heterocyclyl, substituted $C_{3-10}$heterocyclyl, $C_{5-10}$heteroaryl and substituted $C_{5-10}$heteroaryl;

$R_9$ is optionally substituted with 0-1 Rio substituent;

Rio is selected from the group consisting of hydroxyl, halogen, Ci-salkyl, substituted $C_{1-8}$alkyl, $C_{2-8}$alkenyl, substituted $C_{2-8}$alkenyl, $C_{2-8}$alkynyl, substituted $C_{2-8}$alkynyl, $C_{1-8}$alkoxy, substituted $C_{1-8}$alkoxy, $C_{6-10}$aryl, substituted $C_{6-10}$aryl, $C_{3-8}$cycloalkyl, substituted $C_{3-8}$cycloalkyl, $C_{3-10}$heterocyclyl, substituted $C_{3-10}$heterocyclyl, $C_{5-10}$heteroaryl, substituted $C_{5-10}$heteroaryl, -CO-$R_{16}$ and -$(CH_2)_nNR_{11}R_{12}$;

$R_{11}$ or $R_{12}$ is optionally selected from the group consisting of H, Ci-salkyl, substituted Ci-salkyl, $C_{2-8}$alkenyl, substituted $C_{2-8}$alkenyl, $C_{2-8}$ alkynyl, substituted $C_{2-8}$alkynyl, $C_{1-8}$alkoxy, substituted $C_{1-8}$alkoxy, $C_{3-8}$cycloalkyl, substituted $C_{3-8}$cycloalkyl, $C_{6-10}$aryl, substituted $C_{6-10}$aryl, $C_{5-10}$heteroaryl, substituted $C_{5-10}$heteroaryl, $C_{3-10}$heterocyclyl and substituted $C_{3-10}$ heterocyclyl;

$R_{14}$ or $R_{15}$ is optionally selected from the group consisting of H, Ci-salkyl, substituted Ci-salkyl, $C_{2-8}$alkenyl, substituted $C_{2-8}$alkenyl, -CO-$C_{2-8}$alkenyl, substituted -CO-$C_{2-8}$alkenyl, $C_{2-8}$alkynyl, substituted $C_{2-8}$alkynyl, $C_{3-10}$cycloalkyl, substituted $C_{3-10}$cycloalkyl, $C_{6-10}$aryl, substituted $C_{6-10}$aryl, $C_{5-10}$heteroaryl, substituted $C_{5-10}$ heteroaryl, $C_{3-10}$heterocyclyl and substituted $C_{3-10}$ heterocyclyl;

$R_{16}$ is optionally selected from the group consisting of $C_{1-8}$alkyl, substituted $C_{1-8}$ alkyl, $C_{2-8}$alkenyl, substituted $C_{2-8}$alkenyl, $C_{2-8}$alkynyl, substituted $C_{2-8}$alkynyl, $C_{3-10}$cycloalkyl, substituted $C_{3-10}$cycloalkyl, $C_{6-10}$aryl, substituted $C_{6-10}$aryl, $C_{5-10}$heteroaryl, substituted $C_{5-10}$heteroaryl, $C_{3-10}$heterocyclyl, substituted $C_{3-10}$ heterocyclyl and -$NR_{11}R_{12}$;

$R_{17}$ is selected from the group consisting of oxo, $C_{1-8}$alkyl, substituted $C_{1-8}$alkyl, $C_{2-8}$alkenyl, substituted $C_{2-8}$alkenyl, $C_{2-8}$alkynyl, substituted $C_{2-8}$alkynyl, $C_{3-10}$cycloalkyl, substituted $C_{3-10}$cycloalkyl, $C_{6-10}$aryl, substituted $C_{6-10}$ aryl, $C_{5-10}$ heteroaryl, substituted $C_{5-10}$ heteroaryl, $C_{3-10}$ heterocyclyl, substituted $C_{3-10}$ heterocyclyl, or $R_{17}$ is $C_{3-10}$ cycloalkyl, taken together with the carbon atom to which they are attached form a spiro ring;

M is 0 or 1;

N is 0, 1 or 2;

and provided that:

if ring A is a 5-member heteroaryl containing 2 or 3 N atoms, then $R_2$ is vinylamide substituted 6-member heterocyclyl comprising oxygen heteroatom, wherein vinylamide substituted 6-member heterocyclyl optionally substituted with 0-1 $R_{13}$ substituent;

if T is $CR_{17}$, and $R_{17}$ is oxo, then ring A is not a 5-member heteroaryl comprising N heteroatom.

The present invention further provides some preferred technical solutions with regard to the compound of formula (I):

In some embodiments, the L, Q and T of formula (I) are selected from the following groups:

(i) L is C, Q is N, T is $CH_2$;
(ii) L isC, Q is N, T is C;
(iii) L is C, Q is C, T is N;
(iv) L isC, Q isN, T is CH;
(v) L is N, Q is N, T is $CH_2$; or
(vi) L is C, Q is CH, T is O.

In some embodiments, the compound of formula (I) is the compound of formula (II);

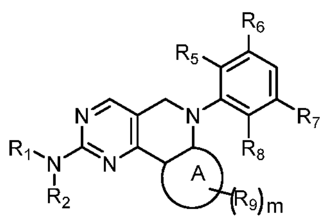

Formula (II)

wherein, the $R_1$, $R_2$, $R_5$, $R_6$, $R_7$, $R_8$, $R_9$ and m have the same definition as formula (I).

In some embodiments, ring A of formula (I) is phenyl, $C_{5-6}$heteroaryl or $C_{10}$heterocyclyl, wherein the $C_{5-6}$heteroaryl optionally containing 1, 2 or 3 heteroatoms selected from N and S, the $C_{10}$heterocyclyl is a fused bicyclic which has two N atoms and one O atom in the ring.

In some embodiments, $R_9$ of formula (I) is selected from the group consisting of H, halogen, cyano, $C_{1-6}$alkyl, halogen substituted $C_{1-6}$alkyl, $-(CH_2)_nNR_{11}R_{12}$ substituted amino, $C_{1-6}$alkoxy which substituted with substituted $C_6$ heterocyclyl, wherein $R_{11}$ and $R_{12}$ are each optionally selected from $C_{1-6}$alkyl.

In some embodiments,

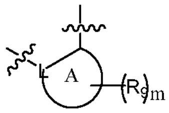

of formula (I) is

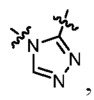, 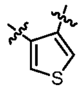, 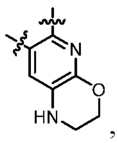, 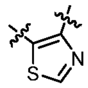,

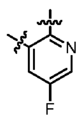, 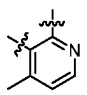, 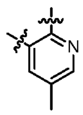, 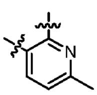, 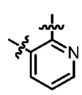, 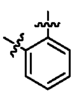, 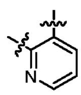, 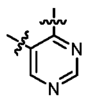, 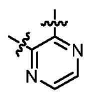, 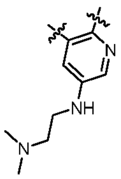, 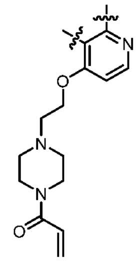,

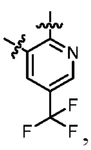, 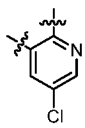, or 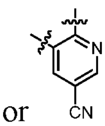.

In some embodiments, $R_1$ of formula (I) is H, $R_2$ is selected from the group consisting of $C_{5-6}$cycloalkyl, substituted $C_{5-6}$cycloalkyl, $C_{5-7}$heterocyclyl, substituted $C_{5-7}$heterocyclyl and phenyl, wherein the $C_{5-7}$heterocyclyl optionally containing 1 or 2 heteroatoms selected from N and O.

In some embodiments, $R_2$ of formula (I) is substituted with 1 or 2 $R_{13}$ substituents, $R_{13}$ is selected from the group consisting of $C_{5-6}$ heterocyclyl, substituted $C_{5-6}$ heterocyclyl, $-NR_{14}R_{15}$ and $-CO-R_{16}$, $R_{14}$ is H, $R_{15}$ is $-CO-C_{2-4}$ alkenyl, $R_{16}$ is $C_{1-3}$alkyl or substituted $C_{1-3}$ alkyl.

In some embodiments, $R_{13}$ of formula (I) is substituted with 0-1 $R_{18}$ substituent, $R_{18}$ is selected from the group consisting of $C_{1-6}$ alkyl, $C_{5-6}$heterocyclyl and substituted $C_{5-6}$ heterocyclyl, wherein the $C_{5-6}$ heterocyclyl containing 1 or 2 heteroatoms selected from N and O.

In some embodiments, $R_2$ of formula (I) is

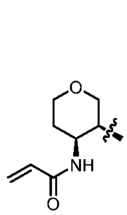, 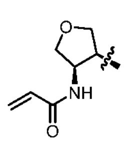, 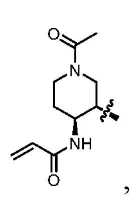, 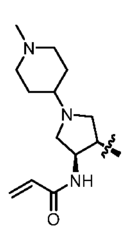,

or 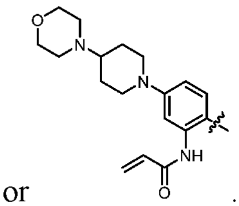 .

In some embodiments, $R_5$, $R_6$, $R_7$ and $R_8$ of formula (I) are each independently selected from the group consisting of hydrogen, halogen, $C_{1-3}$alkoxy and substituted $C_{1-3}$alkoxy.

In some embodiments, $R_5$ and $R_8$ of formula (I) are selected from the following groups:

(i) Both $R_5$ and $R_8$ are chlorine;
(ii) Both $R_5$ and $R_8$ are hydrogen;
(iii) $R_5$ is hydrogen, $R_8$ is chlorine;or
(iv) $R_5$ is chlorine, $R_8$ is hydrogen.

In some embodiments, $R_6$ and $R_7$ of formula (I) are selected from the following groups:

(i) Both $R_6$ and $R_7$ are methoxy;
(ii) $R_6$ is methoxy, $R_7$ is H ;or
(iii) $R_6$ is H, $R_7$ is methoxy.

In some embodiments, m of formula (I) is 0 or 1.

In some embodiments, n of formula (I) is 2.

The present invention further provides a compound or a pharmaceutical acceptable salt thereof, wherein the compound is selected from the group consisting of:

(1) *N*-(2-((6-(2,6-dichloro-3,5-dimethoxyphenyl)-5,6-dihydropyrimido[5,4-*c*][1,8] naphthyridin-2-yl)amino)-5-(4-morpholinopiperidin-1-yl)phenyl)acrylamide;
(2) *N*-((3*S*,4*S*)-3-((6-(2,6-dichloro-3,5-dimethoxyphenyl)-5,6-dihydropyrimido[5,4-*c*][1,8] naphthyridin-2-yl)amino)tetrahydro-2*H*-pyran-4-yl)acrylamide;
(3) *N*-((3*S*,4*S*)-3-((6-(2,6-dichloro-3,5-dimethoxyphenyl)-5-oxo-5,6-dihydropyrimido [5,4-*c*][1,8]naphthyridin-2-yl)amino)tetrahydro-2*H*-pyran-4-yl)acrylamide;
(4) *N*-((3*S*,4*S*)-3-((6-(2,6-dichloro-3,5-dimethoxyphenyl)-5-methyl-5,6-dihydropyrimido [5,4-*c*][1,8]naphthyridin-2-yl)amino)tetrahydro-2*H*-pyran-4-yl)acrylamide;
(5) *N*-((3*S*,4*S*)-3-((6-(2,6-dichloro-3,5-dimethoxyphenyl)-5,6-dihydropyrimido [5,4-*e*][1,2,4]triazolo[4,3-*a*]pyrimidin-2-yl)amino)tetrahydro-2*H*-pyran-4-yl)acrylamide;
(6) *N*-((3*S*,4*S*)-3-((6-(2,6-dichloro-3,5-dimethoxyphenyl)-5,6-dihydropyrimido [5,4-*c*]quinolin-2-yl)amino)tetrahydro-2*H*-pyran-4-yl)acrylamide;
(7) *N*-((3*R*,4*S*)-4-((6-(2,6-dichloro-3,5-dimethoxyphenyl)-5,6-dihydropyrimido [5,4-*c*][1,8]naphthyridin-2-yl)amino)tetrahydrofuran-3-yl)acrylamide;
(8) *N*-((3*S*,4*S*)-3-((6-(2,6-dichloro-3,5-dimethoxyphenyl)-5,6-dihydropyrimido[5,4-*c*][1,5] naphthyridin-2-yl)amino)tetrahydro-2*H*-pyran-4-yl)acrylamide;
(9) *N*-((3*S*,4*S*)-3-((6-(2,6-dichloro-3,5-dimethoxyphenyl)-5,6-dihydropyrido[2,3-*d*:4,5-*d*] dipyrimidin-2-yl)amino)tetrahydro-2*H*-pyran-4-yl)acrylamide;
(10) *N*-((3*S*,4*S*)-3-((6-(2,6-dichloro-3,5-dimethoxyphenyl)-5,6-dihydropyrazino[2',3':5,6] pyrido[4,3-*d*]pyrimidin-2-yl)amino)tetrahydro-2*H*-pyran-4-yl)acrylamide;
(11) *N*-((3*S*,4*S*)-3-((6-(2,6-dichloro-3,5-dimethoxyphenyl)-9-fluoro-5,6-dihydropyrimido [5,4-*c*][1,8]naphthyridin-2-yl)amino)tetrahydro-2*H*-pyran-4-yl)acrylamide;
(12) *N*-((3*S*,4*S*)-3-((6-(2,6-dichloro-3,5-dimethoxyphenyl)-10-methyl-5,6-dihydropyrimido[5,4-*c*][1,8]naphthyridin-2-yl)amino)tetrahydro-2*H*-pyran-4-yl)acrylamide;
(13) *N*-((3*S*,4*S*)-3-((6-(2,6-dichloro-3,5-dimethoxyphenyl)-9-methyl-5,6-dihydropyrimido [5,4-*c*][1,8]naphthyridin-2-yl)amino)tetrahydro-2*H*-pyran-4-yl)acrylamide;
(14) *N*-((3*S*,4*S*)-3-((6-(2-chloro-3,5-dimethoxyphenyl)-9-methyl-5,6-dihydropyrimido [5,4-*c*][1,8]naphthyridin-2-yl)amino)tetrahydro-2*H*-pyran-4-yl)acrylamide;
(15) *N*-((3*S*,4*S*)-3-((6-(2,6-dichloro-3,5-dimethoxyphenyl)-8-methyl-5,6-dihydropyrimido [5,4-*c*][1,8]naphthyridin-2-yl)amino)tetrahydro-2*H*-pyran-4-yl)acrylamide;

(16) *N*-((3*S*,4*S*)-3-((6-(2,6-dichloro-3,5-dimethoxyphenyl)-9-(trifluoromethyl)-5,6-dihydropyrimido[5,4-*c*][1,8]naphthyridin-2-yl)amino)tetrahydro-2*H*-pyran-4-yl)acrylamide;

(17) *N*-((3*S*,4*S*)-3-((6'-(2,6-dichloro-3,5-dimethoxyphenyl)-6'*H*-spiro[cyclopropane-1,5'-pyrimido[5,4-*c*][1,8]naphthyridin]-2'-yl)amino)tetrahydro-2*H*-pyran-4-yl)acrylamide;

(18) *N*-((3*S*,4*S*)-3-((6-(2,6-dichloro-3,5-dimethoxyphenyl)pyrimido[4,5-*f*][1,7] naphthyridin-2-yl)amino)tetrahydro-2*H*-pyran-4-yl)acrylamide;

(19) *N*-((3*S*,4*S*)-3-((6-(2,6-dichloro-3,5-dimethoxyphenyl)-5-methyl-5,6-dihydrothieno [3',4':5,6]pyrido[4,3-*d*]pyrimidin-2-yl)amino)tetrahydro-2*H*-pyran-4-yl)acrylamide;

(20) *N*-((3*S*,4*S*)-3-((6-(2,6-dichloro-3,5-dimethoxyphenyl)-6*H*-pyrido[3',2':4,5]pyrano [3,2-*d*]pyrimidin-2-yl)amino)tetrahydro-2*H*-pyran-4-yl)acrylamide;

(21) *N*-((3*S*,4*S*)-3-((6-(2-chloro-5-methoxyphenyl)-5,6-dihydropyrimido[5,4-*c*][1,8] naphthyridin-2-yl)amino)tetrahydro-2*H*-pyran-4-yl)acrylamide;

(22) *N*-((3*S*,4*S*)-3-((6-(2,6-dichloro-3,5-dimethoxyphenyl)-6,9,10,11-tetrahydro-5*H* -[1,4]oxazino[2,3-b]pyrimido[4,5-*f*][1,8]naphthyridin-2-yl)amino)tetrahydro-2*H*-pyran-4-yl )acrylamide;

(23) *N*-((3*R*,4*S*)-4-((10-(2-(4-acryloylpiperazin-1-yl)ethoxy)-6-(2,6-dichloro-3,5 -dimethoxyphenyl)-5,6-dihydropyrimido[5,4-*c*][1,8]naphthyridin-2-yl)amino)tetrahydrofur an-3-yl)acrylamide;

(24) *N*-((3*R*,4*S*)-1-acetyl-3-((6-(2,6-dichloro-3,5-dimethoxyphenyl)-5,6-dihydropyrimido [5,4-*c*][1,8]naphthyridin-2-yl)amino)piperidin-4-yl)acrylamide;

(25) *N*-((3*S*,4*S*)-3-((4-(2,6-dichloro-3,5-dimethoxyphenyl)-5-methyl-4,5-dihydrothiazolo [5',4':5,6]pyrido[4,3-d]pyrimidin-8-yl)amino)tetrahydro-2*H*-pyran-4-yl)acrylamide;

(26) *N*-((3*S*,4*S*)-3-((6-(2,6-dichloro-3,5-dimethoxyphenyl)-9-((2-(dimethylamino)ethyl) amino)-5,6-dihydropyrimido[5,4-*c*][1,8]naphthyridin-2-yl)amino)tetrahydro-2*H*-pyran-4-yl )acrylamide;

(27) *N*-((3*S*,4*R*)-4-((6-(2,6-dichloro-3,5-dimethoxyphenyl)-5,6-dihydropyrimido [5,4-*c*][1,8]naphthyridin-2-yl)amino)-1-(1-methylpiperidin-4-yl)pyrrolidin-3-yl)acrylamide;

(28) *N*-((3*S*,4*S*)-3-((9-chloro-6-(2,6-dichloro-3,5-dimethoxyphenyl)-5,6-dihydropyrimido[5,4-*c*][1,8]naphthyridin-2-yl)amino)tetrahydro-2*H*-pyran-4-yl)acrylamide;

(29) *N*-((3*S*,4*S*)-3-((9-cyano-6-(2,6-dichloro-3,5-dimethoxyphenyl)-5,6-dihydropyrimido [5,4-*c*][1,8]naphthyridin-2-yl)amino)tetrahydro-2*H*-pyran-4-yl)acrylamide;

(30) *N*-((3*S*,4*S*)-3-((6-(2-chloro-5-methoxyphenyl)-9-methyl-5,6-dihydropyrimido [5,4-*c*][1,8]naphthyridin-2-yl)amino)tetrahydro-2*H*-pyran-4-yl)acrylamide;

(31) *N*-((3*S*,4*S*)-3-((6-(2-chloro-3-methoxyphenyl)-9-methyl-5,6-dihydropyrimido [5,4-*c*][1,8]naphthyridin-2-yl)amino)tetrahydro-2*H*-pyran-4-yl)acrylamide;

(32) *N*-((3*S*,4*S*)-3-((6-(2,6-dichloro-3-methoxyphenyl)-9-methyl-5,6-dihydropyrimido [5,4-*c*][1,8]naphthyridin-2-yl)amino)tetrahydro-2*H*-pyran-4-yl)acrylamide;

(33) *N*-((3*S*,4*S*)-3-((6-(2,6-dichloro-3,5-dimethoxyphenyl)-[1,2,4]triazolo [4',3':1,6]pyrido[2,3-d]pyrimidin-2-yl)amino)tetrahydro-2*H*-pyran-4-yl)acrylamide; or

(34) *N*-(3-((9-chloro-6-(2,6-dichloro-3,5-dimethoxyphenyl)-5-oxo-5,6-dihydropyrimido [5,4-*c*][1,8]naphthyridin-2-yl)amino)tetrahydro-2*H*-pyran-4-yl)acrylamide.

The present invention also provides a pharmaceutical composition comprising a therapeutically effective amount of at least any one of the compounds of formula (I) and at least one pharmaceutically acceptable excipient.

The present invention further provides a pharmaceutically composition in which the weight ratio of the compound of formula (I) and the said excipient is 0.0001-10.

The present invention provides the use of the compound of formula (I) or pharmaceutical composition in the manufacture of a medicament.

The present invention further provides a preferred technical solution for such use:
Preferably, the use is for treating, preventing, delaying or arresting the onset or progression of cancer or cancer metastasis.

Preferably, the use is for manufacturing a medicament for use in the treatment of the disease mediated by FGFR4.

Preferably, the disease is cancer.

Preferably, the cancer is selected from the group consisting of breast cancer, multiple myeloma, bladder cancer, endometrial cancer, gastric cancer, cervical cancer, rhabdomyosarcoma, non-small cell lung cancer, small cell lung cancer, pleomorphic lung cancer, ovarian cancer, esophageal cancer, melanoma, colorectal cancer , Hepatocellular carcinoma, head and neck tumors, hepatobiliary cell carcinoma, myelodysplastic syndrome, malignant glioma, prostate cancer, thyroid cancer, Schwann cell tumor, lung squamous cell carcinoma, lichenoid keratosis, Synovial sarcoma, skin cancer, pancreatic cancer, testicular cancer or liposarcoma.

Preferably, the use is as a FGFR4 inhibitor.

The present invention also provides a method of treating or preventing the disease mediated by FGFR4 by

administering a therapeutically effective amount at least any one of the compounds of Formula (I) or the pharmaceutical composition to a subject.

Preferably, in the above method, the disease mediated by FGFR4 is cancer.

Preferably, in the above method, the cancer is selected from the group consisting of breast cancer, multiple myeloma, bladder cancer, endometrial cancer, gastric cancer, cervical cancer, rhabdomyosarcoma, non-small cell lung cancer, small cell lung cancer, pleomorphic lung cancer, ovarian cancer, esophageal cancer, melanoma, colorectal cancer , Hepatocellular carcinoma, head and neck tumors, hepatobiliary cell carcinoma, myelodysplastic syndrome, malignant glioma, prostate cancer, thyroid cancer, Schwann cell tumor, lung squamous cell carcinoma, lichenoid keratosis, Synovial sarcoma, skin cancer, pancreatic cancer, testicular cancer or liposarcoma.

The present invention also provides a method for treating cancer, which comprises administrating at least any one of the compounds of Formula (I) or the pharmaceutical composition to a subject, the said cancer is breast cancer, multiple myeloma, bladder cancer, endometrial cancer, gastric cancer, cervical cancer, rhabdomyosarcoma, non-small cell lung cancer, small cell lung cancer, pleomorphic lung cancer, ovarian cancer, esophageal cancer, melanoma, colorectal cancer , Hepatocellular carcinoma, head and neck tumors, hepatobiliary cell carcinoma, myelodysplastic syndrome, malignant glioma, prostate cancer, thyroid cancer, Schwann cell tumor, lung squamous cell carcinoma, lichenoid keratosis, Synovial sarcoma, skin cancer, pancreatic cancer, testicular cancer or liposarcoma.

Preferably, in the above method, the subject is human.

Unless otherwise stated, the terms used in the present invention have the following meanings:

The term "alkyl" includes saturated hydrocarbon groups having straight and branched-chain or cyclic moieties. For example, alkyl group includes but not limited to methyl, ethyl, propyl, isopropyl, cyclopropyl, n-butyl, isobutyl, sec-butyl, t-butyl, cyclobutyl, n-pentyl, 3-(2-methyl)butyl, 2-pentyl, 2-methylbutyl, neopentyl, cyclopentyl, n-hexyl, 2-hexyl, 2-methylpentyl and cyclohexyl. Similarly, $C_{1-8}$, as in $C_{1-8}$ alkyl is defined to identify the group as having 1, 2, 3, 4, 5, 6, 7 or 8 carbon atoms in a linear, branched or cyclic arrangement.

"Alkenyl" and "alkynyl" groups include straight, branched-chain or cyclic alkenes and alkynes. Likewise, "$C_{2-8}$ alkenyl" and "$C_{2-8}$ alkynyl" means an alkenyl or alkynyl group having 2, 3, 4, 5, 6, 7 or 8 carbon atoms in linear or branched-chain or cyclic arrangement.

"Alkoxy" refers to the oxygen ethers form of the previously described straight or branched-chain or cyclic alkyl groups.

The term "aryl", as used herein, unless otherwise indicated, refers to an unsubtituted or substituted mono- or polycyclic ring system containing carbon atoms. The preferred aryl is 6-10 membered mono- or bi-cyclic aromatic ring systems. Phenyl and naphthyl are preferred aryls. The most preferred aryl is phenyl.

The term "heteroaryl" refers to a monovalent heteroatom group formed by the removal of one hydrogen atom from a carbon atom of a parent heteroaromatic ring system. The heteroaryl group includes a 5- to 7-membered aromatic, monocyclic ring comprising at least one hetero atom selected from N, O or S, for example, 1 to 4 hetero atoms, or preferably 1 to 3 hetero atoms, and the other atom on the ring is carbon; the polyheteroaryl ring includes at least one hetero atom selected from N, O or S, for example, 1 to 4 hetero atoms, or preferably 1 to 3 hetero atoms, and other atoms on the ring is carbon and at least one of the heteroatoms is on the aromatic ring. Particularly preferred heteroaryl groups are $C_{3-10}$ heteroaryl groups including, but not limited to, pyrrolyl, furyl, thienyl, pyridyl, pyranyl, pyrazolyl, pyrimidinyl, pyridazinyl, pyrazinyl, imidazolyl, thiazolyl, oxazolyl, isoxazolyl, triazolyl, indolyl, benzofuranyl, benzothiazolyl, benzimidazolyl, benzopyrazolyl, benzotriazolyl, carbazolyl, quinolyl, isoquinolinyl, purinyl and the similar groups.

However, in any case, the heteroaryl group and the aryl group do not cross each other or contain each other. Thus, according to the above definition, if at least one all-carbon aromatic ring is fused to a heterocyclic group, a heteroaryl group is obtained instead of an aryl group.

"Cycloalkyl" refers to a saturated or unsaturated cyclic group without aromaticity. According to the particular level of saturation, the terms "cycloalkyl", "cycloalkenyl" or "cycloalkynyl" are employed, respectively. Representative cycloalkyl groups include, but not limited to, cyclopropane, cyclobutane, cyclopentane, cyclohexane or cyclohexene, and the like. Specifically, the cycloalkyl group may be a $C_{3-10}$ cycloalkyl group such as a $C_{3-6}$ cycloalkyl group.

"Heterocyclyl" refers to a saturated or unsaturated cyclic group without aromaticity, and wherein one or more carbon atoms (and the attached hydrogen atoms) can be substituted with the same or different heteroatom and the corresponding hydrogen atom, respectively. Representative heteroatom which substitute the carbon atoms include, but are not limited to, N, P, O, S, and Si. The terms "heterocycloalkyl" or "heterocyclenyl" are used, respectively when it is necessary to describe the particular degree of saturation. Representative heterocyclyl groups include, but are not limited to, epoxy compounds, imidazolidines, morpholine, piperazine, piperidine, pyrazolidine, pyrrolidine, quinuclidine, tetrahydrofuran or tetrahydropyran, and the like. The substituted heterocyclyl group also includes a ring system substituted with at least one oxygen-containing (=O) or oxide (-O-) substituent, such as piperidine-nitrogen-oxide, morpholinyl-nitrogen-oxide, 1-oxo-1-thiomorpholinyl and 1-dioxy-1-thiomorpholinyl.

However, in any case, the heterocycloalkyl group and the cycloalkyl group do not cross each other or contain each other. Thus, according to the above definition, if at least one carbocyclic ring is fused to a heterocycloalkyl group

to form a di-, poly- or spiro-ring, it will still be defined as a heterocycloalkyl group.

Besides, if a heteroaryl is fused to a heterocyclyl to form a di-, poly- or spiro-ring, it will be defined as a heterocyclyl instead of heteroaryl.

"Halogen" refers to fluorine (F), chlorine (Cl), bromine (Br) or iodine (I). Preferred halogen refers to fluorine, chlorine and bromine.

"Halo" refers to a fluoro, chloro, bromo or iodo group. Preferred halo refers to fluoro and chloro.

"Substituted" refers that one or more hydrogen atoms in a group are each substituted with the same or different substituents. Representative substituents include, but are not limited to, halogen, amino, hydroxy, oxo, carbonyl, cyano, alkyl, alkoxy, aryl, cycloalkyl, heterocyclyl, heteroaryl, alkylpiperazine, morpholinyl. In some embodiments, the substituents include, but are not limited to, F, Cl, CN, amino, hydroxyl, cyano, methy, trifluoromethy, cyclopropyl, phenyl, dimethylamino,

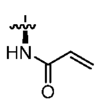, 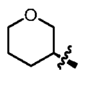, 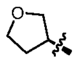, 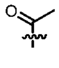, 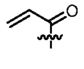, 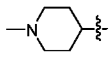, 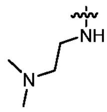, 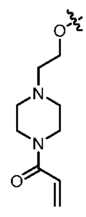, 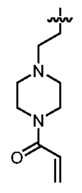,

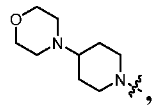, 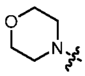 or 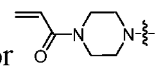.

Whenever, the term "alkyl" or "aryl" or its prefix root appear in the name of a substituent (such as an aralkyl group, or a dialkylamino group), the substituents should be defined according to the aforementioned "alkyl" and "aryl". The specified number of carbon atoms (e.g., $C_{1-6}$) will independently represent the number of carbon atoms in an alkyl moiety or an alkyl moiety in a larger substituent (wherein the alkyl group is the prefix root).

"Compound" as used herein includes a compound of Formula (I), and all pharmaceutically acceptable forms thereof. These pharmaceutically acceptable forms include salts, solvates, non-covalent complexes, chelates or prodrugs thereof, or any mixture of all of the above.

"Pharmaceutically acceptable" means it is well-known for use in animals, particularly for use in humans.

The term "composition" as used in the present invention includes products comprising a specific amount of a particular component, as well as any product derived directly or indirectly from a particular quantity of a particular component. Therefore, a pharmaceutical composition comprising the compound of the present invention as an active ingredient and a method of preparing the same are the contents of the present invention.

"Therapeutically effective amount" means that when a compound is administered to a subject to treat and prevent and/or inhibit at least one clinical condition of a disease, condition, symptom, indication, and/or discomfort, a dose sufficient to produce a certain effect on the treatment of disease, condition, symptom, indication, or discomfort. The specific "effective therapeutic amount" may vary depending on the compound, the route of administration, the age of the patient, the weight of the patient, the type of the disease or discomfort being treated, the symptoms and severity, and the like. Wherever possible, a suitable dosage will be apparent to those skilled in the art and may be determined by routine experimentation.

The compounds of the present invention may also be present in the form of pharmaceutically acceptable salts. For use in medicine, the salts of the compounds of this invention refer to non-toxic "pharmaceutically acceptable salts". The pharmaceutically acceptable salt forms include pharmaceutically acceptable acidic/anionic or basic/cationic salts. The pharmaceutically acceptable acidic/anionic salt generally takes a form in which the basic nitrogen is protonated with an inorganic or organic acid. Representative organic or inorganic acids include hydrochloric, hydrobromic, hydriodic, perchloric, sulfuric, nitric, phosphoric, acetic, propionic, glycolic, lactic, succinic, maleic, fumaric, malic, tartaric, citric, benzoic, mandelic, methanesulfonic, hydroxyethanesulfonic, benzenesulfonic, oxalic, pamoic, 2-naphthalenesulfonic, p-toluenesulfonic, cyclohexanesulfamic, salicyclic, saccharinic or trifluoroacetic. Pharmaceutically acceptable basic/cationic salts include and are not limited to aluminum, calcium, chloroprocaine, choline, diethanolamine, ethylenediamine, lithium, magnesium, potassium, sodium and zinc.

The present invention includes within its scope the prodrugs of the compounds of this invention. In general, such prodrugs will be functional derivatives of the compounds that are readily converted in vivo into the required compound. Thus, in the methods of treatment of the present invention, the term "administering" shall encompass the treatment of the various disorders described with the compound specifically disclosed or with a compound which may not be

specifically disclosed, but which converts to the specified compound in vivo after administration to the subject. Conventional procedures for the selection and preparation of suitable prodrug derivatives are described, for example, in "Design of Prodrugs", ed. H. Bundgaard, Elsevier, 1985.

It is intended that the definition of any substituent or variable at a particular location in a molecule be independent of its definitions elsewhere in that molecule. It is understood that substituents and substitution patterns on the compounds of this invention can be selected by one of ordinary skill in the art to provide compounds that are chemically stable and that can be readily synthesized by techniques know in the art as well as those methods set forth herein.

When the compound of Formula (I) and pharmaceutically acceptable salts thereof exist in the form of solvates or polymorphic forms, the present invention includes any possible solvates and polymorphic forms. A type of a solvent that forms the solvate is not particularly limited so long as the solvent is pharmacologically acceptable. For example, water, ethanol, propanol, acetone or the like can be used.

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. Salts derived from such inorganic bases include aluminum, ammonium, calcium, copper (ic and ous), ferric, ferrous, lithium, magnesium, manganese (ic and ous), potassium, sodium, zinc and the like salts. Particularly preferred are the ammonium, calcium, magnesium, potassium, and sodium salts. Salts derived from pharmaceutically acceptable organic non-toxic bases include salts of primary, secondary, and tertiary amines, as well as cyclic amines and substituted amines such as naturally occurring and synthesized substituted amines. Other pharmaceutically acceptable organic non-toxic bases from which salts can be formed include ion exchange resins such as, for example, arginine, betaine, caffeine, choline, *N'*,*N'*-dibenzylethylenediamine, diethylamine, 2-diethylaminoethanol, 2-dimethylaminoethanol, ethanolamine, ethylenediamine, *N*-ethylmorpholine, *N*-ethylpiperidine, glucamine, glucosamine, histidine, hydrabamine, isopropylamine, lysine, methylglucamine, morpholine, piperazine, piperidine, polyamine resins, procacine, purines, theobromine, triethylamine, trimethylamine, tripropylamine, tromethamine and the like.

When the compound of the present invention is basic, its corresponding salt can be conveniently prepared from pharmaceutically acceptable non-toxic acids, including inorganic and organic acids. Such acids include, for example, acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Preferred acids are citric, hydrobromic, formic, hydrochloric, maleic, phosohoric, sulfuric and tartaric acids. Particularly preferred acids are formic and hydrochloric acid. Since the compounds of Formula (I) are intended for pharmaceutical use they are preferably provided in substantially pure form, for example at least 60% pure, more suitably at least 75% pure, especially at least 98% pure (% are on a weight for weight basis).

The pharmaceutical compositions of the present invention comprise a compound represented by Formula I (or a pharmaceutically acceptable salt thereof) as an active ingredient, a pharmaceutically acceptable carrier and optionally other therapeutic ingredients or adjuvants. The compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administrated. The pharmaceutical compositions may be conveniently presented in unit dosage form and prepared by any of the methods well known in the art of pharmacy.

In practice, the compounds represented by Formula I, or a prodrug, or a metabolite, or pharmaceutically acceptable salts thereof, of this invention can be combined as the active ingredient in intimate admixture with a pharmaceutical carrier according to conventional pharmaceutical compounding techniques. The carrier may take a wide variety of forms depending on the form of preparation desired for administarion, e.g., oral or parenteral (including intravenous). Thus, the pharmaceutical compositions of the present invention can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as an oil-in-water emulsion, or as a water-in-oil liquid emulsion. In addition to the common dosage forms set out above, the compound represented by Formula I, or a pharmaceutically acceptable salt thereof, may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

Thus, the pharmaceutical composition of this invention may include a pharmaceutically acceptable carrier and a compound, or a pharmaceutically acceptable salt, of Formula I. The compounds of Formula I, or a pharmaceutically acceptable salt thereof, can also be included in pharmaceutical compositions in combination with one or more other therapeutically active compounds.

The pharmaceutical carrier employed can be, for example, a solid, liquid, or gas. Examples of solid carriers include lactose, gypsum powder, sucrose, talc, gelatin, agar, pectin, acacia, magnesium stearate, stearic acid, mannitol, sorbitol, microcrystalline cellulose, inorganic salts, starch, pregelatinized starch, powdered sugar and the like. Examples of liquid carriers are sugar syrup, peanut oil, olive oil, and water. Examples of gaseous carriers include carbon dioxide and nitrogen. In preparing the compositions for oral dosage form, any convenient pharmaceutical media may be employed. For example, water, glycols, oils, alcohols, flavoring agents, preservatives, coloring agents, and the like may be used to form oral liquid preparations such as suspensions, elixirs and solutions; while carriers such as starches, sugars, microcrustalline cellulose, diluents, granulating agents, lubricants, binders, disintegrating agents, and the like may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques.

A tablet containing the compounds or composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a lubricant, inert diluent, surface active or dispersing agent. Molded tablets may be made by molding in a suitable machine, a mixture of the powdered compound or composition moistened with an inert liquid diluent. Each tablet preferably contains from about 0.01mg to about 5g of the active ingredient and each cachet or capsule preferably containing from about 0.1mg to about 0.5g of the active ingredient. For example, a formulation intended for the oral administration to humans may contain from about 0.1mg to about 0.5g of active agent, compounded with an appropriate and convenient amount of carrier material which may vary from about 5 to about 99.99 percent of the total composition. Unit dosage forms will generally contain between from about 0.1mg to about 0.5g of the active ingredient, typically 0.1mg, 0.2mg, 0.5mg, 1mg, 2mg, 2.5mg, 5mg, 10mg, 25mg, 50mg, 100mg, 200mg, 300mg, 400mg or 500mg.

Pharmaceutical compositions of the present invention suitable for parenteral administration may be prepared as solutions or suspensions of the active compounds in water. A suitable surfactant can be included such as sodium lauryl sulfate, polysorbate-80 (Tween-80), polyoxyethylene hydrogenated castor oil, poloxamer. Dispersions can also be prepared in glycerol, liquid polyethylene glycols, and mixtures thereof in oils. Further, a preservative can be included to prevent the detrimental growth of microorganisms.

Pharmaceutical compositions of the present invention suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacterial and fungi. The carrier can be a solvent or dispersion medium containing, for example, water, ethanol, polyol (e.g., glycerol, propylene glycol and liquid polyethylene glycol), vegetable oils, and suitable mixtures thereof.

Pharmaceutical compositions of the present invention can be in a form suitable for topical use such as, for example, an aerosol, cream, ointment, lotion, dusting powder, or the like. Further, the compositions can be in a form suitable for use in transdermal devices. These formulations may be prepared, utilizing a compound represented by Formula I of this invention, or a pharmaceutically acceptable salt thereof, via conventional processing methods. As an example, a cream or ointment is prepared by admixing hydrophilic material and water, together with about 5 wt% to about 50wt% of the compound, to produce a cream or ointment having a desired consistency.

Pharmaceutical compositions of this invention can be in a form suitable for rectal administration wherein the carrier is a solid. It is preferable that the mixture forms unit dose suppositories. Suitable carriers include cocoa butter and other materials commonly used in the art. The suppositories may be conveniently formed by first admixing the composition with the softened or melted carriers followed by chilling and shaping in molds.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including antioxidants) and the like. Furthermore, other adjuvants can be included to render the formulation isotonic with the blood of the intended recipient. Compositions containing a compound described by Formula I, or a pharmaceutically acceptable salts thereof, may also be prepared in powder or liquid concentrate form.

## EXAMPLES

To be clearer, the present invention is further exemplified by the following examples. The following examples are intended to illustrate the specific embodiments of the present invention to make those skilled in art understand the prenent invention, but not to limit the protection scope of the present invention. In the examples of the present invention, the techniques or methods, unless expressly stated otherwise, are conventional techniques or methods in the art.

Unless otherwise indicated, all parts and percentages of the invention are calculated by weight, the temperatures

are given in degrees Celsius (°C).

The following abbreviations have been used in the examples:

ATP: Adenine nucleoside triphosphate;
$(Boc)_2O$: Di-tert-butyl dicarbonate;
DCM: Dichloro methane;
DIPEA: *N*,*N*-diisopropylethylamine;
DMAP: 4-dimethylaminopyridine;
DMF: *N*,*N*-dimethylformamide;
DMSO: Dimethy sulfoxide;
EA: Ethyl acetate;
HCOOEt: Ethyl formate;
HOAc: Acetic acid;
KOAc: Potassium acetate;
LCMS or LC-MS: Liquid Chromatograph Mass Spectrometer;
m-CPBA or mCPBA:M-chloroperoxybenzoic acid;
MeMgBr: Methy Magnesium Bromide;
MeOH: Methanol;
$PdCl_2(dppf)CH_2Cl_2$:[1,1'-Bis(diphenylphosphine)ferrocene]dichloro palladium dichloro methane complex;
$Pd(OAc)_2$:Palladium(II) acetate;
$Pd(PPh_3)_4$:Tetra (triphenylphosphine)palladium;
rt, r.t. or RT:Room temperature;
h, hr or hrs:hour;
TEA: Triethylamine;
THF: Tetrahydrofuran;
TLC: Thin layer chromatography;
Xant-phos: 4,5-Bis(diphenylphosphino)-9,9-dimethylxanthene.

**Example 1: Preparation of *N*-(2-((6-(2,6-dichloro-3,5-dimethoxyphenyl)-5,6-dihydropyrimido[5,4-*c*][1,8] naphthyridin-2-yl)amino)-5-(4-morpholinopiperidin-1-yl)phenyl)acrylamide**

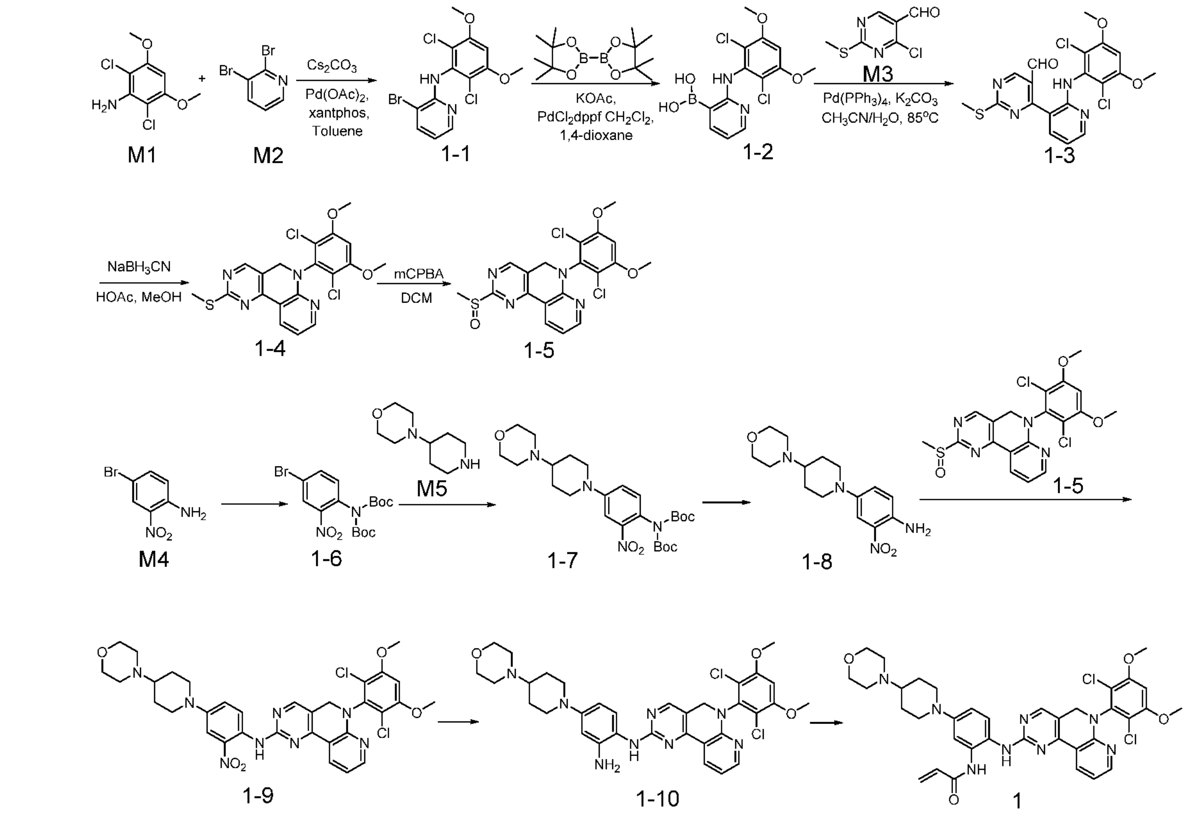


M1 M2 1-1 1-2 M3 1-3

1-4 1-5

M4 1-6 M5 1-7 1-8 1-5

1-9 1-10 1

**Step1: Preparation of compound 1-1**

M1 (0.85g), M2 (0.91g), $Cs_2CO_3$ (2.49g), $Pd(OAc)_2$(0.09g), Xant-phos (0.44g) were dissolved in toluene (40mL), and reacted at 115°C for 5 hrs under $N_2$ protection. LCMS showed the reaction was completed. Concentrated under reduced pressure, the residue was dissolved in water, extracted with DCM, the organic layer was washed with water, saturated brine successively, concentrated, the residue was purified by column chromatography eluting with hexane:dichloromethane = 1:5 to obtain 1.28g compound 1-1 as a yellow solid.
LC-MS [M+H$^+$] 376.9.

**Step2: Preparation of compound 1-2**

Compound 1-1 (1.25g), pinacol diborate (0.91g), KOAc(1.20g) and $PdCl_2(dppf)CH_2Cl_2$(0.14g) were dissolved in 1,4-dioxane(40mL), and reacted at 100 °C for 15hrs under $N_2$ protection. LCMS showed the reaction was completed. Concentrated under reduced pressure, the residue was dissolved in water, extracted with EA, the organic layer was washed with water, saturated brine successively, concentrated, the residue was purified by column chromatography eluting with dichloromethane:methonal = 30:1 to obtain 1.07g compound 1-2 as a brown solid.
LC-MS [M+H$^+$] 343.0.

**Step3: Preparation of compound 1-3**

Compound 1-2(1.03g), M3(0.57g), $K_2CO_3$(0.83g) and $Pd(PPh_3)_4$ (0.35g) were dissolved in 35 mL of acetonitrile/12mL of water, and reacted at 85 °C for 4.5hrs under $N_2$ protection. LCMS showed the reaction was completed. Concentrated under reduced pressure, the residue was dissolved in water, extracted with DCM, the organic layer was washed with water, saturated brine successively, concentrated, the residue was purified by column chromatography eluting with dichloromethane:methonal = 200:1 to obtain compound 1-3(0.93g) as a yellow solid.
LC-MS [M+H$^+$] 451.0.

**Step4: Preparation of compound 1-4**

Compound 1-3 (0.92g), acetic acid (0.19g) and cyano sodium borohydride (0.20g) were dissolved in methonal (25mL), and reacted at room temperature for 14hrs under $N_2$ protection. LCMS showed the reaction was completed.

Concentrated under reduced pressure, the residue was dissolved in water, saturated $Na_2CO_3$ aqueous solution and DCM, extracted with DCM twice, the organic layer was combined and washed with water and saturated brine successively, concentrated,the residue was purified by column chromatography eluting with DCM:$CH_3OH$ = 100:1 to obtain compound 1-4 (0.39g) as a yellow solid.
LC-MS [M+H$^+$] 434.9.

**Step5: Preparation of compound 1-5**

Compound 1-4 (0.35g) was dissolved in DCM (25 mL), mCPBA(85%) (0.36g) was added slowly under an ice-water bath condition, after the addition, warmed to room temperature naturally and reacted for 7hrs. LCMS showed the reaction was completed.The reaction solution was washed with saturated $NaHCO_3$ aqueous solution twice, the organic layers were combined, washed with water and saturated brine, dried over anhydrous $Na_2SO_4$ for 1 hr, filtered and concentrated under reduced pressure to obtain compound 1-5 (0.39g) as an orange-yellow solid.
LC-MS [M+H$^+$] 451.0.

**Step6: Preparation of compound 1-6**

To a solution of compound M4 (10.85g), DMAP (1.22g), triethylamine (17.4mL) in tetrahydrofuran (250mL), $(Boc)_2O$(24.01g) dissolved in tetrahydrofuran(40mL) was added slowly under stirring, after the addition, the reaction was refluxed for 16hrs under $N_2$ protection. TLC showed the reaction was almost completed. Concentrated under reduced pressure, the residue was dissolved in water, extracted with DCM, the organic was washed with water, saturated brine successively, concentrated, the residue was purified by column chromatography eluting with hexane:ethyl acetate = 5:1 to obtain compound 1-6 (20.18g) as a yellow solid.
LC-MS [M+H$^+$] 417.1.

**Step7: Preparation of compound 1-7**

Compound 1-6 (3.02g), M5 (1.13g), $Cs_2CO_3$ (5.36g), $Pd_2dba_3$ (0.60g), Xant-phos (0.76g) were dissolved in toluene (50mL), and reacted at 110°C for 14hrs under $N_2$ protection. LCMS showed the reaction was completed. Concentrated under reduced pressure, the residue was dissolved in water, extracted with DCM, the organic was washed with water, saturated brine successively, concentrated, the residue was purified by column chromatography eluting with DCM:MeOH = 40:1 to obtain compound 1-7 (2.20g) as a red-brown sticky substance.
LC-MS [M+H$^+$] 507.1.

**Step8: Preparation of compound 1-8**

To a solution of compound 1-7(2.20g) in DCM (30mL), trifluoroacetate (20mL) was added slowly, reacted at room temperature for 2 hrs, the pH of the residue was adjusted to 8~9 using saturated $Na_2CO_3$ aqueous solution, extracted with DCM, washed with water and saturated brine , the organic layer was dried over anhydrous $Na_2SO_4$ for 1 hr, filtered and concentrated under reduced pressure to obtain crude product(1.20g) as red-brown solid which was used for the next step directly without purification.
LC-MS [M+H$^+$] 307.1.

**Step9: Preparation of compound 1-9**

Compound 1-8 (307mg), compound1-5(451mg) were dissolved in anhydrous DMF(10mL), potassium tert-butoxide (169mg) was added slowly at -10°C, after the addition, the mixture was reacted for 2 hrs after warming to room temperature naturally. LCMS showed the reaction was completed. The reaction was quenched with water, extracted with EA, the organic layer was washed with water and saturated brine successively, concentrated, the residue was purified by column chromatography eluting with dichloromethane:methanol = 40:1 to obtain compound 1-9 (200mg) as a yellow solid.
LC-MS [M+H$^+$] 693.1.

**Step10: Preparation of compound 1-10**

The solution of compound 1-9 (0.20g), reduced iron powder (0.13g) and ammonium chloride (0.13g) in ethanol (30mL) and water (6mL) was refluxed for 3 hrs under $N_2$ protection. LCMS showed the reaction was completed. The reaction mixture was filtered without cooling, filtrate was concentrated under reduced pressure, the residue was purified

by column chromatography eluting with DCM: MeOH = 9:1 to obtain compound 1-10 (0.16g) as an orange-yellow solid.
LC-MS [M+H+] 663.1.

**Step11: Preparation of compound 1**

To a solution of compound 1-10 (100mg) in DCM (8mL) was added trimethylamine (0.1mL), and then acrylic chloride was added slowly at -10°C, the reaction was reacted at such temperature for 1h. LCMS showed the reaction was almost completed, the mixture was quenched with saturated $NaHCO_3$ aqueous solution, the DCM layer was washed with water and saturated brine successively, concentrated under reduced pressure, the residue was purified by preparative TLC eluting with DCM:MeOH = 10:1 to obtain compound 1(43mg) as an orange-yellow solid.
LC-MS [M+H+] 717.2.

**Example 2: Preparation of *N*-((3*S*,4*S*)-3-((6-(2,6-dichloro-3,5-dimethoxyphenyl)-5,6-dihydropyrimido[5,4-*c*][1,8]naphthyridin-2-yl)amino)tetrahydro-2*H*-pyran-4-yl)acrylamide**

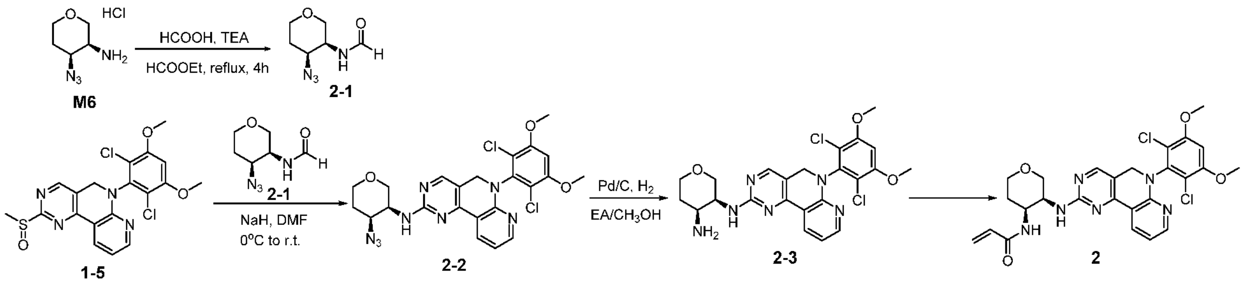

M6 2-1 1-5 2-1 2-2 2-3 2

**Step1: Preparation of compound 2-1**

M6(3.57g), TEA(6.2mL) and formic acid (88%)(2.09g) were dissolved in ethyl formate (100mL), the reaction was refluxed for 4 hrs. TLC showed the reaction was completed. Concentrated under reduced pressure, the residue was dissolved in ethyl acetat, the organic layer was washed with a little water and saturated brine successively, concentrated to obtain compound 2-1 (3.49g) as an off-white solid which was used in the next reaction directly without purification.

**Step2: Preparation of compound 2-2**

To a solution of compound 2-1(90mg) in anhydrous DMF (2mL), NaH (60%) (42mg) was added slowly under an ice-salt bath condition, the reaction was reacted at such temperature for 40 mins, and then compound 1-5 (239mg) was added, after the addition, the reaction was warmed to rt and reacted for 40 mins. LCMS showed the reaction was completed. The reaction mixture was quenched with water, the organic layer was washed with water and saturated brine successively, concentrated, the residue was purified by column chromatography eluting with $DCM:CH_3OH=40:1$ to obtain compound 2-2 (139mg) as an orange-yellow sticky substance.
LC-MS [M+H+] 529.1.

**Step3: Preparation of compound 2-3**

A mixture of compound 2-2(135mg) and 10% Pd/C(50mg) in $EA/CH_3OH$(15mL/10mL), was reacted at 30°C for 2hrs under $H_2$ condition. Filtered, concentrated under reduced pressure to obtain crude product of compound 2-3 (132mg) as an orange-yellow sticky substance which was used in the next reaction directly without purification.
LC-MS [M+H+] 503.1.

**Step4: Preparation of compound 2**

To a solution of compound 2-3(130mg) in DCM (8mL), trimethylamine (0.12mL) was added, and acrylic chloride (23mg) was added slowly at -20°C, the reaction was reacted at such temperature for 1h. LCMS showed the reaction was completed, quenched with saturated $NaHCO_3$ aqueous solution, the DCM layer was washed with water and saturated brine successively, concentreated under reduced pressure, the residue was purified by thick preparative TLC eluting

with DCM/MeOH=40:1 to obtain compound 2 (41 mg) as a light yellow solid.
LC-MS [M+H+] 557.1.

**Example 3: Preparation of *N*-((3*S*,4*S*)-3-((6-(2,6-dichloro-3,5-dimethoxyphenyl)-5-oxo-5,6-dihydropyrimido [5,4-*c*][1,8]naphthyridin-2-yl)amino)tetrahydro-2*H*-pyran-4-yl)acrylamide**

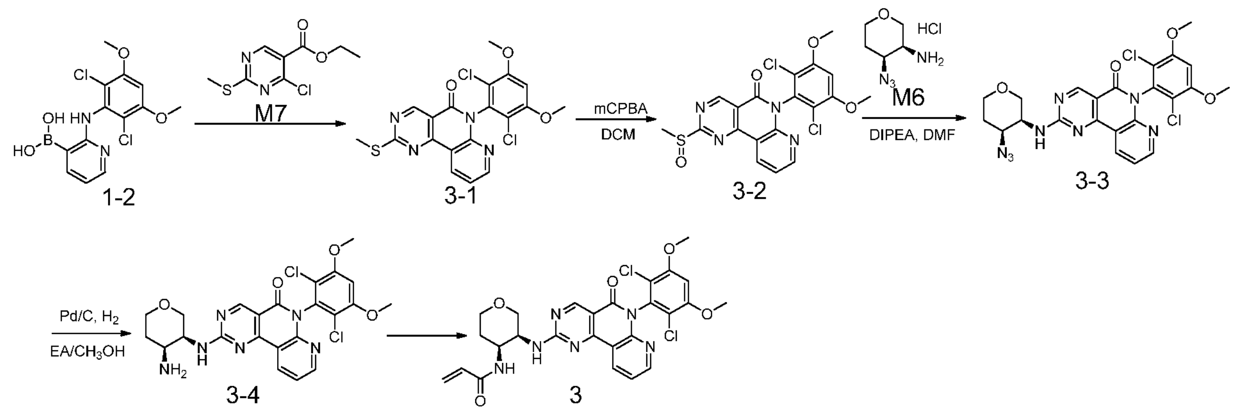


1-2, 3-1, 3-2, 3-3, 3-4, 3

**Step1: Preparation of compound 3-1**

Compound 3-1 was prepared using a similar method shown in step 3 of Example 1, 4-chloro-2-methylthiopyrimidine-5-ethyl carboxylate (M7) replaced M3 as the original material to prepared compound 3-1.
LC-MS [M+H+] 449.0.

**Step2: Preparation of compound 3-2**

Compound 3-2 was prepared using a similar method described in step 5 of Example 1.
LC-MS [M+H+] 464.9.

**Step3: Preparation of compound 3-3**

Compound 3-2 (780mg), M6 (300mg), DIPEA (1.26mL) and DMF (8mL) was added into the sealed tube successively, and reacted at 80°C for 2hrs. Quenched with water, extracted with EA, washed with saturated brine four times, concentrated, and purified by column chromatography eluting with DCM/MeOH= 100:1 to obtain compound 3-3 as an orangr-yellow solid.
LC-MS [M+H+] 543.0.

**Step4: Preparation of compound 3-4**

Compound 3-4 was prepared using a similar method described in step 3 of Example 2.
LC-MS [M+H+] 517.1.

**Step5: Preparation of compound 3**

Compound 3 was prepared using a similar method described in step4 of Example 2.
LC-MS [M+H+] 571.5.

**Example 4: Preparation of *N*-((3*S*,4*S*)-3-((6-(2,6-dichloro-3,5-dimethoxyphenyl)-5-methyl-5,6-dihydropyrimido [5,4-*c*][1,8]naphthyridin-2-yl)amino)tetrahydro-2*H*-pyran-4-yl)acrylamide**

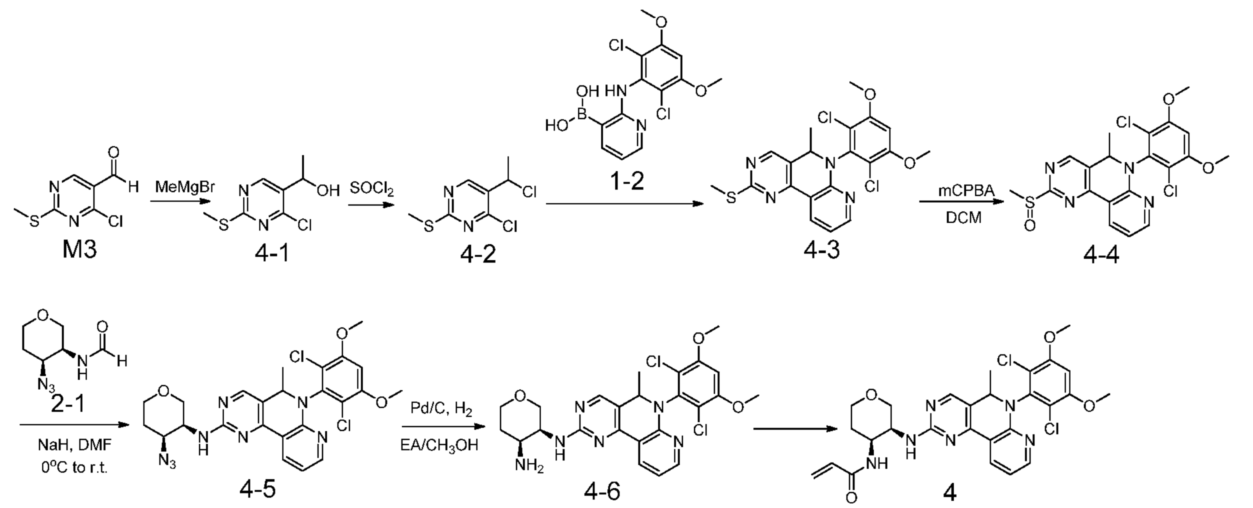


**Step1: Preparation of compound 4-1**

M3 (3.77g) was dissolved in anhydrous THF (100mL), methyl magnesium bromide (1M in THF) (1.2 mL) was added slowly in an ice-water bath (<5°C) condition, and continued reacting for 1h at the same tempereature. LCMS showed the reaction was completed. The reaction was quenched with ammonium chloride aqueous solution, extracted with DCM, the organic layer was washed with water and saturated brine successively, concentrated, the residue was purified by column chromatography eluting with DCM:$CH_3OH$=50:1 to obtain compound 4-1 (3.72g) as a yellow liquid.
LC-MS [$M+H^+$] 205.4.

**Step2: Preparation of compound 4-2**

Compound 4-1(2.05g) was dissolved in dichloromethane (20mL), thionyl chloride (3.63 mL) was added slowly in an ice-water bath (<5°C) condition, after the addition, continued reacting for 2 hrs at the same tempereature. LCMS showed the reaction was completed. The reaction solution was added into a stirring ice-water mixture (1L), extracted with dichloromethane twice, the organic layers were combined, washed with water and saturated brine successively, the residue was purified by column chromatography eluting with hexane: dichloromethane=3:1 to obtain compound 4-2 as a light yellow liquid.
LC-MS [$M+H^+$] 223.3.

**Step3: Preparation of compound 4-3**

Compound 4-3 was prepared using a similar method described in step 3 of Example 1, compound 4-2 replaced M3 as the original material.
LC-MS [$M+H^+$] 449.0.

**Step4: Preparation of compound 4-4**

Compound 4-4 was prepared using a similar method described in step 5 of Example 1.
LC-MS [$M+H^+$] 465.0.

**Step5: Preparation of compound 4-5**

Compound 4-5 was prepared using a similar method described in step 2 of Example 2.
LC-MS [$M+H^+$] 543.0.

**Step6: Preparation of compound 4-6**

Compound 4-6 was prepared using a similar method described in step 3 of Example 2.
LC-MS [$M+H^+$] 517.1.

**Step7: Preparation of compound 4**

Compound 4 was prepared using a similar method described in step 4 of Example 2. LC-MS [M+H$^+$] 571.1.

The compounds in Table 1 can be prepared using a similar method described in aforementioned examples with the different original materials and the appropriate reagents. For example, when M2 in Example 1 is replaced by

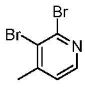, 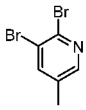, 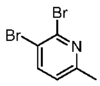, 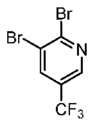, 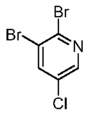

or

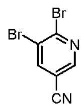,

the compound 12, 13, 15, 16, 28 or 29 can be prepared with reference to the preparation method of Example 2.

Table 1

| Ex.NO. | Chemical Structure | Chemical Name | LC-MS [M+H$^+$] |
|---|---|---|---|
| 5 | 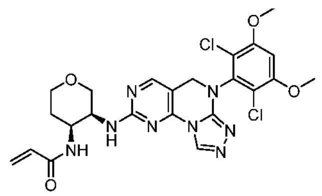 | *N*-((3*S*,4*S*)-3-((6-(2,6-dichloro-3,5-dimethoxy phenyl)-5,6-dihydropyrimido[5,4-*e*][1,2,4]tria zolo[4,3-a] pyrimidin-2-yl)amino)tetrahydro-2 *H*-pyran-4-yl) acrylamide | 547.1 |
| 6 | 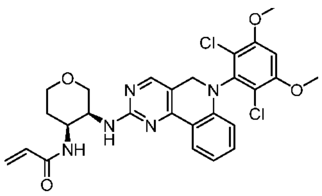 | *N*-((3*S*,4*S*)-3-((6-(2,6-dichloro-3,5-dimethoxy phenyl)-5,6-dihydropyrimido[5,4-c]quinolin-2 -yl)amino) tetrahydro-2*H*-pyran-4-yl)acrylami de | 556.1 |
| 7 | 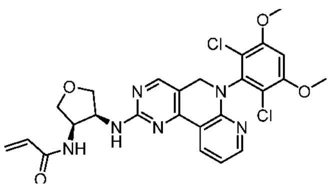 | *N*-((3*R*,4*S*)-4-((6-(2,6-dichloro-3,5-dimethoxy phenyl)-5,6-dihydropyrimido[5,4-*c*][1,8]napht hyridin-2-yl)amino) tetrahydrofuran-3-yl)acryl amide | 543.7 |
| 8 | 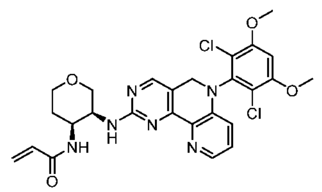 | *N*-((3*S*,4*S*)-3-((6-(2,6-dichloro-3,5-dimethoxy phenyl)-5,6-dihydropyrimido[5,4-*c*][1,5]napht hyridin-2-yl)amino) tetrahydro-2*H*-pyran-4-yl) acrylamide | 557.1 |

(continued)

| Ex.NO. | Chemical Structure | Chemical Name | LC-MS [M+H+] |
|---|---|---|---|
| 9 | 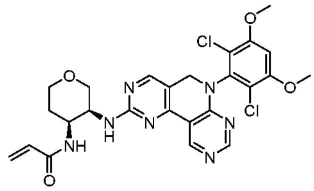 | *N*-((3*S*,4*S*)-3-((6-(2,6-dichloro-3,5-dimethoxy phenyl)-5,6-dihydropyrido[2,3-*d*:4,5-*d'*]dipyri midin-2-yl)amino) tetrahydro-2*H*-pyran-4-yl)a crylamide | 558.0 |
| 10 | 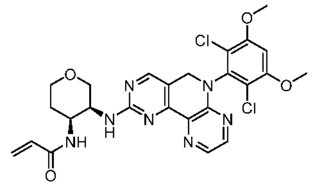 | *N*-((3*S*,4*S*)-3-((6-(2,6-dichloro-3,5-dimethoxy phenyl)-5,6-dihydropyrazino [2',3':5,6]pyrido[ 4,3-d]pyrimidin-2-yl)amino)tetrahydro-2*H*-py ran-4-yl)acrylamide | 558.0 |
| 11 | 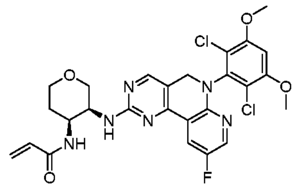 | *N*-((3*S*,4*S*)-3-((6-(2,6-dichloro-3,5-dimethoxy phenyl)-9-fluoro-5 ,6-dihydropyrimido [5,4-*c*][ 1,8]naphthyridin-2-yl)amino)tetrahydro-2*H*-p yran-4-yl)acrylamide | 575.7 |
| 12 | 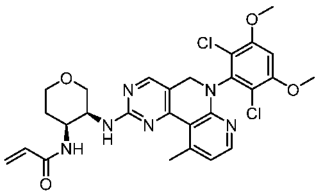 | *N*-((3*S*,4*S*)-3-((6-(2,6-dichloro-3,5-dimethoxy phenyl)-10-methyl-5,6-dihydropyrimido[5,4-*c* ][1,8]naphthyridin-2-yl)amino)tetrahydro-2*H*-pyran-4-yl)acrylamide | 571.7 |
| 13 | 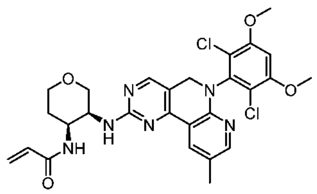 | *N*-((3*S*,4*S*)-3-((6-(2,6-dichloro-3,5-dimethoxy phenyl)-9-methyl-5 ,6-dihydropyrimido [5,4-*c*][ 1,8]naphthyridin-2-yl)amino)tetrahydro-2*H*-p yran-4-yl)acrylamide | 571.7 |
| 14 | 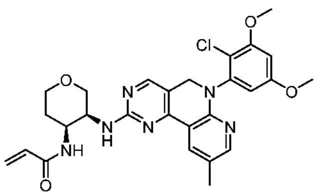 | *N*-((3*S*,4*S*)-3-((6-(2-chloro-3,5-dimethoxyphe nyl)-9-methyl-5,6-dihydropyrimido[5,4-*c*] [1,8 ]naphthyridin-2-yl)amino)tetrahydro-2*H*-pyra n-4-yl)acrylamide | 537.7 |
| 15 | 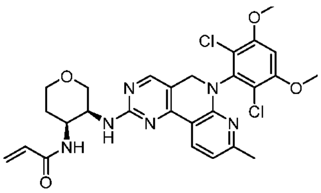 | *N*-((3*S*,4*S*)-3-((6-(2,6-dichloro-3,5-dimethoxy phenyl)-8-methyl-5 ,6-dihydropyrimido [5,4-*c*][ 1,8]naphthyridin-2-yl)amino)tetrahydro-2*H*-p yran-4-yl)acrylamide | 571.7 |

(continued)

| Ex.NO. | Chemical Structure | Chemical Name | LC-MS [M+H+] |
|---|---|---|---|
| 16 | 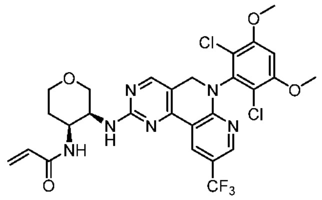 | *N*-((3*S*,4*S*)-3-((6-(2,6-dichloro-3,5-dimethoxy phenyl)-9-(trifluoromethyl)-5,6-dihydropyrimi do[5,4-*c*] [1,8] naphthyridin-2-yl)amino)tetrahy dro-2*H*-pyran-4-yl) acrylamide | 625.6 |
| 17 | 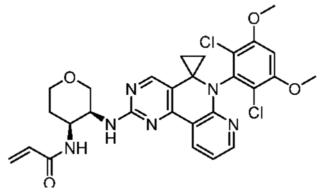 | *N*-((3*S*,4*S*)-3-((6'-(2,6-dichloro-3,5-dimethoxy phenyl)-6*H*-spiro[cyclopropane-1,5'-pyrimido [5,4-*c*][1,8] naphthyridin]-2'-yl)amino)tetrahyd ro-2*H*-pyran-4-yl) acrylamide | 583.1 |
| 18 | 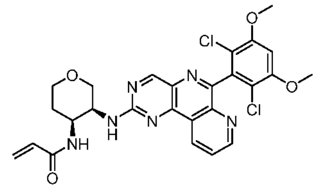 | *N*-((3*S*,4*S*)-3-((6-(2,6-dichloro-3,5-dimethoxy phenyl) pyrimido[4,5-f][1,7]naphthyridin-2-yl) amino)tetrahydro-2*H*-pyran-4-yl)acrylamide | 555.1 |
| 19 | 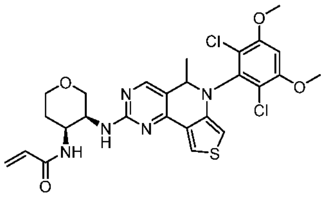 | *N*-((3*S*,4*S*)-3-((6-(2,6-dichloro-3,5-dimethoxy phenyl)-5-methyl-5,6-dihydrothieno[3',4':5,6] pyrido[4,3-*d*] pyrimidin-2-yl)amino)tetrahydro -2*H*-pyran-4-yl) acrylamide | 576.2 |
| 20 | 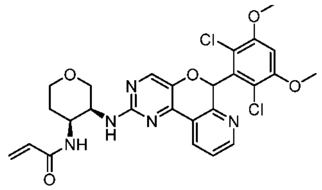 | *N*-((3*S*,4*S*)-3-((6-(2,6-dichloro-3,5-dimethoxy phenyl)-6*H*-pyrido[3',2':4,5]pyrano[3,2-*d*]pyri midin-2-yl)amino) tetrahydro-2*H*-pyran-4-yl)a crylamide | 558.1 |
| 21 | 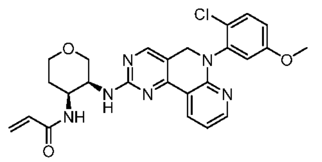 | *N*-((3*S*,4*S*)-3-((6-(2-chloro-5-methoxyphenyl)-5,6-dihydropyrimido[5,4-*c*][1,8]naphthyridin-2-yl)amino ) tetrahydro-2*H*-pyran-4-yl)acrylam ide | 493.1 |
| 22 | 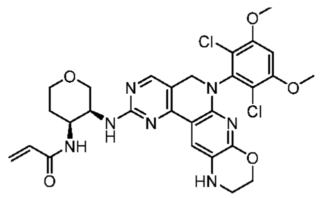 | *N*-((3*S*,4*S*)-3-((6-(2,6-dichloro-3,5-dimethoxy phenyl)-6,9,10,11 -tetrahydro-5*H*-[1,4]oxazino [2,3-b]pyrimido [4,5-*f*][1,8]naphthyridin-2-yl)a mino)tetrahydro-2*H*-pyran-4-yl)acrylamide | 614.1 |

(continued)

| Ex.NO. | Chemical Structure | Chemical Name | LC-MS $[M+H^+]$ |
|---|---|---|---|
| 23 | 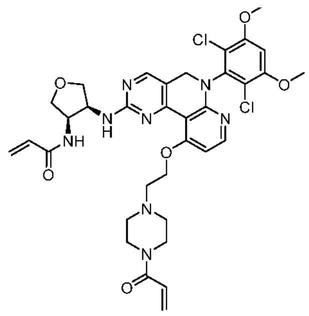 | *N*-((3*R*,4*S*)-4-((10-(2-(4-acryloylpiperazin-1-y l)ethoxy)-6-(2,6-dichloro-3,5-dimethoxypheny 1)-5,6-dihydropyrimido[5,4-*c*][1,8]naphthyridi n-2-yl)amino) tetrahydrofuran-3-yl)acrylamide | 725.2 |
| 24 | 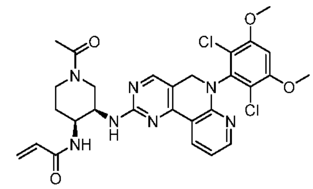 | *N*-((3*R*,4*S*)-1-acetyl-3-((6-(2,6-dichloro-3,5-di methoxyphenyl)-5 ,6-dihydropyrimido [5,4-*c*][ 1,8] naphthyridin-2-yl)amino)piperidin-4-yl)ac rylamide | 598.1 |
| 25 | 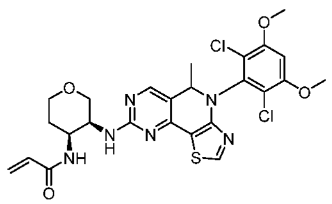 | *N*-((3*S*,4*S*)-3-((4-(2,6-dichloro-3,5-dimethoxy phenyl)-5-methyl-4,5-dihydrothiazolo[5',4':5,6 ]pyrido[4,3-*d*] pyrimidin-8-yl)amino)tetrahydr o-2*H*-pyran-4-yl) acrylamide | 577.1 |
| 26 | 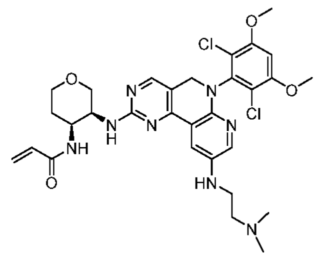 | *N*-((3*S*,4*S*)-3-((6-(2,6-dichloro-3,5-dimethoxy phenyl)-9-((2-(dimethylamino)ethyl)amino)-5, 6-dihydropyrimido [5,4-*c*][1,8]naphthyridin-2-yl)amino)tetrahydro-2*H*-pyran-4-yl)acrylamid e | 643.2 |
| 27 | 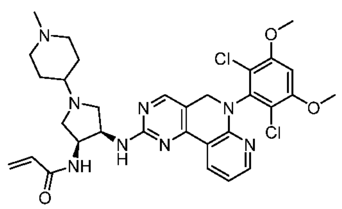 | *N*-((3*S*,4*R*)-4-((6-(2,6-dichloro-3,5-dimethoxy phenyl)-5,6-dihydropyrimido[5,4-*c*][1,8]napht hyridin-2-yl) amino)-1-(1-methylpiperidin-4-yl )pyrrolidin-3-yl) acrylamide | 639.2 |
| 28 | 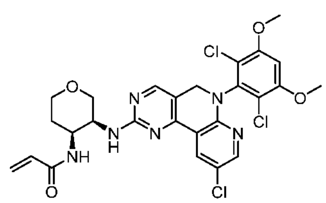 | *N*-((3*S*,4*S*)-3-((9-chloro-6-(2,6-dichloro-3,5-di methoxyphenyl)-5,6-dihydropyrimido [5,4-*c*][ 1,8] naphthyridin-2-yl)amino)tetrahydro-2*H*-p yran-4-yl) acrylamide | 591.6 |

(continued)

| Ex.NO. | Chemical Structure | Chemical Name | LC-MS [M+H+] |
|---|---|---|---|
| 29 | 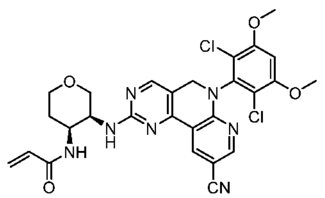 | *N*-((3*S*,4*S*)-3-((9-cyano-6-(2,6-dichloro-3,5-di methoxyphenyl)-5,6-dihydropyrimido [5,4-*c*][ 1,8] naphthyridin-2-yl)amino)tetrahydro-2*H*-p yran-4-yl) acrylamide | 582.8 |
| 30 | 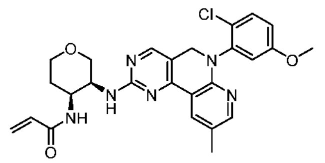 | *N*-((3*S*,4*S*)-3-((6-(2-chloro-5-methoxyphenyl)-9-methyl-5,6-dihydropyrimido[5,4-*c*][1,8]nap hthyridin-2-yl)amino) tetrahydro-2*H*-pyran-4-y l)acrylamide | 507.2 |
| 31 | 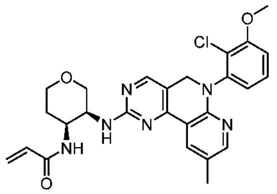 | *N*-((3*S*,4*S*)-3-((6-(2-chloro-3-methoxyphenyl)-9-methyl-5,6-dihydropyrimido[5,4-*c*][1,8]nap hthyridin-2-yl)amino) tetrahydro-2*H*-pyran-4-y l)acrylamide | 507.2 |
| 32 | 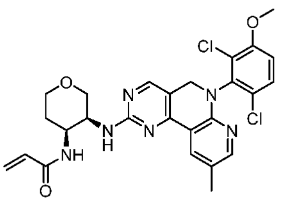 | *N*-((3*S*,4*S*)-3-((6-(2,6-dichloro-3-methoxyphe nyl)-9-methyl-5,6-dihydropyrimido[5,4-*c*] [1,8 ]naphthyridin-2-yl)amino)tetrahydro-2*H*-pyra n-4-yl)acrylamide | 541.1 |
| 33 | 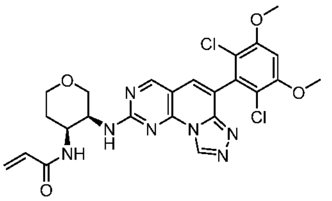 | *N*-((3*S*,4*S*)-3-((6-(2,6-dichloro-3,5-dimethoxy phenyl)-[1,2,4]triazolo[4',3':1,6]pyrido[2,3-*d*] pyrimidin-2-yl)amino)tetrahydro-2*H*-pyran-4-yl)acrylamide | 543.1 |
| 34 | 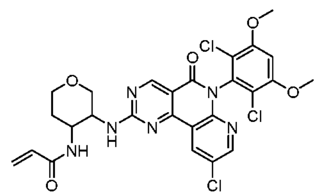 | *N*-(3-((9-chloro-6-(2,6-dichloro-3,5-dimethox yphenyl)-5-oxo-5,6-dihydropyrimido[5,4-*c*][1, 8]naphthyridin-2-yl) amino)tetrahydro-2*H*-pyr an-4-yl)acrylamide | 605.9 |

The NMR data of compounds 13, 14, 16, 28 and 32 are as follows:

$^1$H NMR (DMSO-*d6*, 500MHz) δ(ppm) 8.14 (s, 1H), 8.12 (s, 1H), 7.99 (d, *J* = 7.8 Hz, 1H), 7.78 (s, 1H), 6.96 (s, 1H), 6.57 (d, *J* = 7.5 Hz, 1H), 6.19 (br, 1H), 6.05-6.01 (m, 1H), 5.49 (br, 1H), 4.76 (d, *J*= 14.0 Hz, 1H), 4.71 (d, *J* = 14.0 Hz, 1H), 4.43 (br, 1H), 4.27-4.20 (m, 1H), 3.95 (s, 6H), 3.91-3.72 (m, 2H), 3.68-3.57 (m, 1H), 3.56-3.44 (m, 1H), 2.17 (s, 3H), 2.02-1.85 (m, 1H), 1.71-1.54 (m, 1H). (Compound 13) ;

$^1$H NMR ($CDCl_3$, 500MHz) δ(ppm) 8.14 (s, 1H), 8.03 (s, 1H), 7.96 (s, 1H), 7.04 (d, *J*= 5.0 Hz, 1H), 6.53 (s, 1H), 6.51 (s, 1H), 6.18 (d, *J*= 17.1 Hz, 1H), 5.90 (dd, *J*= 16.7, 10.9 Hz, 1H), 5.80 (d, *J* = 8.0 Hz, 1H), 5.49 (d, *J*= 10.1 Hz, 1H), 4.81 (s, 2H), 4.45 (s, 1H), 4.30 - 4.25 (m, 1H), 4.07-4.01 (m, 1H), 3.98 (d, *J* = 11.7 Hz, 1H), 3.90 (s, 3H), 3.80 (s, 3H), 3.73 (d, *J* = 11.8 Hz, 1H), 3.61 (t, *J* = 12.0 Hz, 1H), 2.21 (s, 3H), 2.08 (d, *J* = 12.4 Hz, 1H), 1.87 (tt, *J* = 12.3, 6.3 Hz, 1H). (Compound 14) ;

$^1$H NMR ($CDCl_3$, 500MHz) δ(ppm) 8.51 (s, 1H), 8.26 (s, 1H), 8.06 (s, 1H), 6.64 (s, 2H), 6.17 (d, *J* = 17.0 Hz, 1H), 5.91

(t, *J* = 10.9 Hz, 2H), 5.51 (d, *J* = 10.1 Hz, 1H), 4.87 (d, *J* = 15.9 Hz, 2H), 4.49 (br, 1H), 4.30-4.24(m, 2H), 4.01-3.95(m, 1H),3.97 (s, 6H), 3.73 (d, *J* = 11.7 Hz, 1H), 3.60 (t, *J*= 11.7 Hz, 1H), 2.14-1.96 (m, 1H), 1.90-1.80 (m, 1H) (Compound 16) ;
$^1$H NMR ($CDCl_3$, 500MHz) δ(ppm) 8.27 (s, 1H), 8.04 (s, 1H), 7.94 (s, 1H), 6.72 (br, 1H), 6.62 (s, 1H), 5.91 (dd, *J*= 16.6 Hz, 10.5Hz, 1H), 5.11 (d, *J*= 8.5 Hz, 1H), 5.53 (d, *J*= 10.0 Hz, 1H), 4.87 (s, 2H), 4.47 (br, 1H), 4.31-4.24(m, 1H), 4.01-3.93(m, 2H), 3.96 (s, 6H), 3.72 (d, *J* = 12.0 Hz, 1H), 3.60 (t, *J*= 12.0 Hz, 1H), 2.07-2.01 (m, 1H), 1.87-1.82 (m, 1H) (Compound 28) ;
$^1$H NMR ($CDCl_3$, 500MHz) δ(ppm) 9.49 (s, 1H), 8.16 (s, 1H), 8.06 (s, 1H), 7.73 (s, 1H), 7.41 (d, *J* = 9.0 Hz, 1H), 6.95 (d, *J* = 8.9 Hz, 2H), 6.22 (d, *J* = 17.0 Hz, 1H), 6.06 (dd, *J* = 17.0, 10.4 Hz, 1H), 5.61 (d, *J*= 10.3 Hz, 1H), 4.88-4.72 (m, 2H), 4.60-4.45 (m, 2H), 4.31 (d, *J*= 11.5 Hz, 1H), 4.20-4.00 (m, 1H), 3.98 (s, 3H), 3.69 (d, *J* = 12.3 Hz, 1H), 3.58 (t, *J* = 11.3 Hz, 1H), 2.29 (s, 3H), 2.26-1.95(m, 1H), 1.84-1.77 (m, 1H). (Compound 32) .

**Pharmacological test**

**Example A: Kinase assay**

The effect of the compounds of the present invention on the activity of tyrosine kinase FGFR4 was evaluated with in vitro kinase detection experiment.The mobility shift assay was used in the experiment, and a fluorescently labeled polypeptide was used as the substrate, the substrate was transformed into a product under the action of the enzyme in the reaction system , and its charge has also changed accordingly. This method can use the difference between the charge of the substrate and the product to separate them, and then detect them separately.

Experiment procedure:

(1) Compound preperation:

DMSO solution of compound (300μM) was diluted to a 100-fold final concentration of DMSO solution in a 384-well plate, 3-fold dilution, 250nL of the compound with 100-fold final concentration was transferred to the target plate OptiPlate-384F by a dispenser Echo 550. The final concentration of the compound were 3000nM, 1000nM, 333.3nM, 111.1nM, 37.04nM, 12.35nM, 4.115nM, 1.372nM, 0.4572nM, 0.1524nM, the compound and the enzyme were incubated for 60 mins;

(2) Kinase reaction:

Prepared 1× kinase buffer, used the 1× kinase buffer to prepare 2.5 fold final concentration of kinase solution, 10 μL of 2.5 fold final concentration kinase solution was added to the compound well and the positive control well respectively, and 10 μL of 1× kinase buffer was added to the negative control well. After centrifugation, the reaction plate was shaken and mixed and incubated at room temperature for 60 mins, a mixture of 25/15 fold final concentration of adenosine triphosphate (ATP) and kinase substrate 22 was prepared with 1 × kinase buffer. 15μL of 25/15fold final concentration of ATP and substrate mixed solution was added to start the reaction, 384-well plate was centrifuged, mixed, and then incubated at room temperature for 30 minutes, 30 μL of the stop detection solution was added to stop the kinase reaction, and the conversion rate was readed with Caliper EZ Reader after centrifugation and mixing;

(3) Data analysis:

$$\text{Calculation formula: inhibition rate\%} = \frac{Conversion\%_{max} - Conversion\%_sample}{Conversion\%_{max} - Conversion\%_min} * 100$$

wherein: Conversion%_sample was the conversion rate of the sample; Conversion%_min was the average value of the negative control well, which represent the conversion rate of the well without enzyme activity; Conversion%_max was the average value of the positive control well, which represent the conversion rate of the well without compound inhibition.

Take the log value of the concentration as the X axis, the percentage inhibition rate as the Y axi, and use GraphPad Prism's log(inhibitor) vs. response-Variable slope(four parameters) of the analysis software to fit the dose-response curve to obtain the ICso value of each compound on the enzyme. The formula is as follows:Y=Bottom+(Top-Bottom)/(1+10 ^ ((Log$IC_{50}$-X)*HillSlope)).

The ICso data of some Examples and BLU554 are shown in Table 2.

Table 2

| Compound number | $IC_{50}$(nM) of compound | |
|---|---|---|
| | FGFR1 | FGFR4 |
| Compound 1 | /① | 9.4 |
| Compound 2 | 912 | 10 |
| Compound 11 | 1531 | 1.8 |
| Compound 13 | 367 | 1.0 |
| Compound 14 | 1435 | 2.6 |
| Compound 15 | 1001 | 5.2 |
| Compound 16 | 166 | 3.6 |
| Compound 28 | 741 | 1.8 |
| BLU554② | 1480 | 13 |
| Note①"/" represents to not tested;(2)BLU554 is the No.40 compound which disclosed by Blueprint Medicines Corporation in WO2015061572. | | |

The compound of the present invention has a great inhibitory effect on FGFR4 kinase, and such compounds have a much stronger inhibitory effect on FGFR4 than that on FGFR1, which represent a great selectivity.

**Example B: Cell proliferation assay**

In vitro cell assay was used to measure the effects of the compound of the present invention on the proliferation of human liver cancer cells Hep3B cells. The CELL TITER-GLO (CTG) luminescent was used as detection method in the assay, which can detect the number of living cells by quantitatively determining ATP. Because ATP participates in a variety of enzymatic reactions in organisms, it is an indicator of the metabolism of living cells, and its content directly reflects the number and cell state of cells, during the experiment, add CellTiter-Glo™ reagent to the cell culture medium to measure the luminescence, the luminescence value is directly proportional to the amount of ATP, and ATP is positively related to the number of living cells, so cell viability can be inspected by detecting the ATP content.

Experiment procedure:

(1) Cell plating:

Take a bottle of Hep3B cells in logarithmic growth phase, the cells were counted after digestion and resuspention, and then were adjusted the cell density and seeded at 180μL per well (1500 cells/well) into a 96-well plate, the plate was incubated for 24hrs in 37°C, 5%$CO_2$ incubator;

(2) Cell drug delivery:

The 600μM test substance dissolved in DMSO was diluted with DMSO in a 1:3 ratio to a 200-fold final concentration solution, then the cell culture medium was diluted 20 fold (10×), and 20μL of the compound solution was added to the 96 wells containing cells in the plate, the final concentration of the compound from high to low was 3000nM, 1000nM, 333.3nM, 111.1nM, 37.04nM, 12.35nM, 4.115nM, 1.372nM, 0.4572nM, and the well plate was placed in a 37°C, 5%$CO_2$ incubator for 96hrs ;

(3) CTG detection:

After 96hrs of incubation, 60μL of CellTiter-Glo® Luminescent Cell Viability Assay solution was added to each well, gently shaked for 2mins, continued incubating for 10mins at room temperature, and the luminescence value of each well on the multifunctional microplate reader was readed.

(4) Data analysis:

Calculated the inhibition rate base on the luminous value,

Inhibition rate %=（blank group value - administration group value）/（blank group value – apoptosis group value）*100

The log value of the concentration was used as the X axis, and the percentage inhibition rate was used as the Y axis. Log (inhibitor) vs. response-Variable slope (four parameters) of GraphPad Prism's was used to fit the dose-response curve, and the $IC_{50}$ of the compound to inhibit cell proliferation was calculated.

The experimental data were shown in Table 3.

Table 3

| Compound number | $IC_{50}$(nM) of the compound on Hep3B cells |
|---|---|
| BLU554 | 62.7 |
| Compound 1 | 6.9 |
| Compound 3 | >3000 |
| Compound 11 | 10.6 |
| Compound 13 | 5.1 |
| Compound 14 | 18.7 |
| Compound 16 | 14.5 |
| Compound 28 | 9.9 |
| Compound 33 | 524.4 |
| Compound 34 | 1027.0 |

The preferred compound of the present invention has a good inhibitory effect on the proliferation of Hep3B cells.

**Example C: Xenograft tumor models**

BALB/c nu/nu female mice were inoculated subcutaneously in the right anterior scapula of $5\times10^6$ human hepatocarcinoma cells Hep3B, and the volume ratio of cell suspension to matrigel was 1:1 (0.2/mL/mouse). The mice were grouped according to tumor size until the average tumor volume was $158mm^3$. The treatment group was given a test compound solution prepared with an appropriate solvent, and the solvent control group was given a blank solvent. During the treatment, the tumor volume was measured twice a week, and the tumor weight was measured after the last dose to determine the compound activity. The tumor growth inhibition rate (%, TGI) was calculated by comparing the tumor volume and weight of the treatment group and the solvent control group. Body weight measurement, as a routine determination of toxicity, has the same frequency as the tumor volume measurement. In this model, compound of example 13 showed a good anti-tumor activity. For example, when the doses were 50mg/kg, 100mg/kg and 200mg/kg (BID×14), the inhibitory rate of Example13 compound on tumor volume growth of Hep3B were 73.02%, 86.26% and 90.26% respectively, the inhibitory rate of tumor weight growth of HepB were 84.76%, 92.27% and 98.15% respectively, which shows that compound of Example13 showed a dose-dependent effect in inhibiting tumor volume and weight. The compound of example 16 also showed a good tumor activity in this model, when the dose was 50mg/kg (BID×14), the inhibitory rates of inhibiting tumor volume and weight growth were 78.37% and 83.85%, respectively. In addition, during the entire experiment, the animals given Example13 and Example16 compounds did not show obvious weight losses, which indicate that, the two compounds are well tolerated under the conditions of the treatment doses.

Although the present invention has been fully described through its embodiments, it is worth noting that various changes and modifications are obvious to those skilled in the art. Such changes and modifications should be included in the range of the appended claims of the present invention.

**Claims**

**1.** A compound of Formula (I) or a pharmaceutically acceptable salt, solvate, chelate, non-covalent complex, or prodrug thereof,

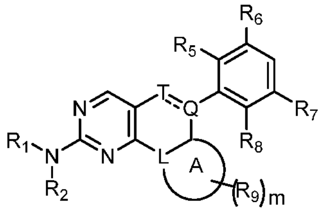

Formula (I)

--- is a single bond or a double bond;
L, Q or T is each independently selected from the group consisting of O, N, C, CH, $CH_2$ and $CR_{17}$;
ring A is $C_{6-10}$ aryl, substituted $C_{6-10}$ aryl, $C_{5-10}$ heteroaryl, substituted $C_{5-10}$ heteroaryl, $C_{5-10}$ heterocyclyl, substituted $C_{5-10}$ heterocyclyl; wherein the $C_{5-10}$ heteroaryl or $C_{5-10}$ heterocyclyl optionally containing 1, 2 or 3 heteroatoms independently selected from N, O and S;
$R_1$ is selected from the group consisting of hydrogen, halogen, $C_{1-8}$ alkyl, substituted $C_{1-8}$ alkyl, $C_{1-8}$ alkoxy, substituted $C_{1-8}$ alkoxy, $C_{2-8}$ alkenyl, substituted $C_{2-8}$ alkenyl, $C_{2-8}$ alkynyl and substituted $C_{2-8}$ alkynyl;
$R_2$ is selected from the group consisting of $C_{3-10}$ cycloalkyl, substituted $C_{3-10}$ cycloalkyl, $C_{3-10}$ heterocyclyl, substituted $C_{3-10}$ heterocyclyl, $C_{6-10}$ aryl, substituted $C_{6-10}$ aryl, $C_{6-10}$ heteroaryl and substituted $C_{6-10}$ heteroaryl, wherein the $C_{3-10}$ heterocyclyl or $C_{6-10}$ heteroaryl optionally containing 1 or 2 heteroatoms selected from N and O ;
$R_2$ is optionally substituted with 1-2 $R_{13}$ substituents;
$R_{13}$ is selected from the group consisting of hydroxyl, halogen, $C_{1-8}$ alkyl, substituted $C_{1-8}$ alkyl, $C_{2-8}$ alkenyl, substituted $C_{2-8}$ alkenyl, $C_{2-8}$ alkynyl, substituted $C_{2-8}$ alkynyl, $C_{1-8}$ alkoxy, substituted $C_{1-8}$ alkoxy, $C_{6-10}$ aryl, substituted $C_{6-10}$ aryl, $C_{3-10}$ cycloalkyl, substituted $C_{3-10}$ cycloalkyl, $C_{3-10}$ heterocyclyl, substituted $C_{3-10}$ heterocyclyl, $C_{5-10}$ heteroaryl, substituted $C_{5-10}$ heteroaryl, $-NR_{14}R_{15}$ and $-CO-R_{16}$, wherein the $C_{3-10}$ heterocyclyl or $C_{5-10}$ heteroaryl optionally containing 1, 2 or 3 heteroatoms selected from N and O;
$R_{13}$ is optionally substituted with 0-1 $R_{18}$ substituent;
$R_{18}$ is selected from the group consisting of hydroxyl, halogen, $C_{1-8}$ alkyl, substituted $C_{1-8}$ alkyl, $C_{2-8}$ alkenyl, substituted $C_{2-8}$ alkenyl, $C_{2-8}$ alkynyl, substituted $C_{2-8}$ alkynyl, $C_{1-8}$ alkoxy, substituted $C_{1-8}$ alkoxy, $C_{6-10}$ aryl, substituted $C_{6-10}$ aryl, $C_{3-10}$ cycloalkyl, substituted $C_{3-10}$ cycloalkyl, $C_{3-10}$ heterocyclyl, substituted $C_{3-10}$ heterocyclyl, $C_{5-10}$ heteroaryl and substituted $C_{5-10}$ heteroaryl, wherein the $C_{3-10}$ heterocyclyl or $C_{5-10}$ heteroaryl containing 1, 2 or 3 heteroatoms selected from N and O;
$R_5$, $R_6$, $R_7$ and $R_8$ is independently selected from the group consisting of hydroxyl, halogen, $C_{1-8}$ alkoxy, substituted $C_{1-8}$ alkoxy, $C_{1-8}$ alkyl, substituted $C_{1-8}$ alkyl, $C_{2-8}$ alkenyl, substituted $C_{2-8}$ alkenyl, $C_{2-8}$ alkynyl, substituted $C_{2-8}$ alkynyl, $C_{3-8}$ cycloalkyl, substituted $C_{3-8}$ cycloalkyl, $C_{6-10}$ aryl, substituted $C_{6-10}$ aryl, $C_{5-10}$ heteroaryl, substituted $C_{5-10}$ heteroaryl, $C_{3-10}$ heterocyclyl and substituted $C_{3-10}$ heterocyclyl;
$R_9$ is selected from the group consisting of H, halogen, amino, cyano, $C_{1-8}$ alkyl, substituted $C_{1-8}$ alkyl, $C_{1-8}$ alkoxy, substituted $C_{1-8}$ alkoxy, $C_{2-8}$ alkenyl, substituted $C_{2-8}$ alkenyl, $C_{2-8}$ alkynyl, substituted $C_{2-8}$ alkynyl, $C_{6-10}$ aryl, substituted $C_{6-10}$ aryl, $C_{3-8}$ cycloalkyl, substituted $C_{3-8}$ cycloalkyl, $C_{3-10}$ heterocyclyl, substituted $C_{3-10}$ heterocyclyl, $C_{5-10}$ heteroaryl and substituted $C_{5-10}$ heteroaryl;
$R_9$ is optionally substituted with 0-1 Rio substituent;
$R_{10}$ is selected from the group consisting of hydroxyl, halogen, $C_{1-8}$ alkyl, substituted $C_{1-8}$ alkyl, $C_{2-8}$ alkenyl, substituted $C_{2-8}$ alkenyl, $C_{2-8}$ alkynyl, substituted $C_{2-8}$ alkynyl, $C_{1-8}$ alkoxy, substituted $C_{1-8}$ alkoxy, $C_{6-10}$ aryl, substituted $C_{6-10}$ aryl, $C_{3-8}$ cycloalkyl, substituted $C_{3-8}$ cycloalkyl, $C_{3-10}$ heterocyclyl, substituted $C_{3-10}$ heterocyclyl, $C_{5-10}$ heteroaryl, substituted $C_{5-10}$ heteroaryl, $-CO-R_{16}$ and $-(CH_2)_nNR_{11}R_{12}$;
$R_{11}$ or $R_{12}$ is optionally selected from the group consisting of H, $C_{1-8}$ alkyl, substituted $C_{1-8}$ alkyl, $C_{2-8}$ alkenyl, substituted $C_{2-8}$ alkenyl, $C_{2-8}$ alkynyl, substituted $C_{2-8}$ alkynyl, $C_{1-8}$ alkoxy, substituted $C_{1-8}$ alkoxy, $C_{3-8}$ cycloalkyl, substituted $C_{3-8}$ cycloalkyl, $C_{6-10}$ aryl, substituted $C_{6-10}$ aryl, $C_{5-10}$ heteroaryl, substituted $C_{5-10}$ heteroaryl, $C_{3-10}$ heterocyclyl and substituted $C_{3-10}$ heterocyclyl;
$R_{14}$ or $R_{15}$ is optionally selected from the group consisting of H, $C_{1-8}$ alkyl, substituted $C_{1-8}$ alkyl, $C_{2-8}$ alkenyl, substituted $C_{2-8}$ alkenyl, $-CO-C_{2-8}$ alkenyl, substituted $-CO-C_{2-8}$ alkenyl, $C_{2-8}$ alkynyl, substituted $C_{2-8}$ alkynyl, $C_{3-10}$ cycloalkyl, substituted $C_{3-10}$ cycloalkyl, $C_{6-10}$ aryl, substituted $C_{6-10}$ aryl, $C_{5-10}$ heteroaryl, substituted $C_{5-10}$

heteroaryl, $C_{3-10}$ heterocyclyl and substituted $C_{3-10}$ heterocyclyl;

$R_{16}$ is optionally selected from the group consisting of $C_{1-8}$ alkyl, substituted $C_{1-8}$ alkyl, $C_{2-8}$ alkenyl, substituted $C_{2-8}$ alkenyl, $C_{2-8}$ alkynyl, substituted $C_{2-8}$ alkynyl, $C_{3-10}$ cycloalkyl, substituted $C_{3-10}$ cycloalkyl, $C_{6-10}$ aryl, substituted $C_{6-10}$ aryl, $C_{5-10}$ heteroaryl, substituted $C_{5-10}$ heteroaryl, $C_{3-10}$ heterocyclyl, substituted $C_{3-10}$ heterocyclyl and $-NR_{11}R_{12}$;

$R_{17}$ is selected from the group consisting of oxo, $C_{1-8}$ alkyl, substituted $C_{1-8}$ alkyl, $C_{2-8}$ alkenyl, substituted $C_{2-8}$ alkenyl, $C_{2-8}$ alkynyl, substituted $C_{2-8}$ alkynyl, $C_{3-10}$ cycloalkyl, substituted $C_{3-10}$ cycloalkyl, $C_{6-10}$ aryl, substituted $C_{6-10}$ aryl, $C_{5-10}$ heteroaryl, substituted $C_{5-10}$ heteroaryl, $C_{3-10}$ heterocyclyl, substituted $C_{3-10}$ heterocyclyl, or $R_{17}$ is $C_{3-10}$ cycloalkyl, taken together with the carbon atom to which they are attached form a spiro ring;

M is 0 or 1;

N is 0, 1 or 2;

and provided that:

if ring A is a 5-member heteroaryl containing 2 or 3 N atoms, then $R_2$ is vinylamide substituted 6-member heterocyclyl comprising oxygen heteroatom, wherein vinylamide substituted 6-member heterocyclyl optionally substituted with 0-1 $R_{13}$ substituent;

if T is $CR_{17}$, and $R_{17}$ is oxo, then ring A is not a 5-member heteroaryl comprising N heteroatom.

**2.** The compound or a pharmaceutically acceptable salt, solvate, chelate, non-covalent complex, or prodrug thereof of claim 1, wherein L, Q and T is selected from the following groups:

(i) L is C, Q is N, T is $CH_2$;
(ii) L isC, Q is N, T is C;
(iii) L is C, Q is C, T is N;
(iv) L isC, Q isN, T is CH;
(v) L is N, Q is N, T is $CH_2$; or
(vi) L is C, Q is CH, T is O.

**3.** The compound or a pharmaceutically acceptable salt, solvate, chelate, non-covalent complex, or prodrug thereof of claim 1, wherein the compound is shown as formula (II):

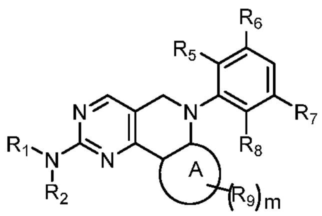

Formula (II).

**4.** The compound or a pharmaceutically acceptable salt, solvate, chelate, non-covalent complex, or prodrug thereof of any one of claims 1-3, wherein ring A is phenyl, $C_{5-6}$ heteroaryl or $C_{10}$ heterocyclyl, wherein the $C_{5-6}$ heteroaryl optionally containing 1, 2 or 3 heteroatoms selected from N and S, the $C_{10}$ heterocyclyl is a fused bicyclic which has two N atoms and one O atom in the ring.

**5.** The compound or a pharmaceutically acceptable salt, solvate, chelate, non-covalent complex, or prodrug thereof of any one of claims 1-3, wherein ring A is $C_6$ heteroaryl.

**6.** The compound or a pharmaceutically acceptable salt, solvate, chelate, non-covalent complex, or prodrug thereof of any one of claims 1-3, wherein $R_9$ is selected from the group consisting of H, halogen, cyano, $C_{1-6}$ alkyl, halogen substituted $C_{1-6}$ alkyl, $-(CH_2)_nNR_{11}R_{12}$ substituted amino, $C_{1-6}$ alkoxy which substituted with substituted $C_6$ heterocyclyl, wherein $R_{11}$ and $R_{12}$ are each optionally selected from $C_{1-6}$ alkyl.

**7.** The compound or a pharmaceutically acceptable salt, solvate, chelate, non-covalent complex, or prodrug thereof of any one of claims 1-3, wherein

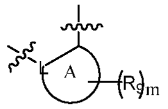 is 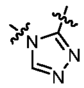, 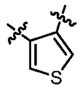,

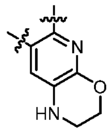, 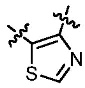, 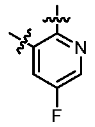, 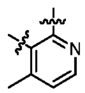, 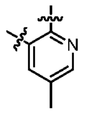, 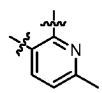, 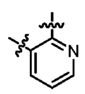, 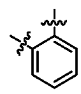, 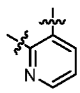, 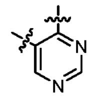, 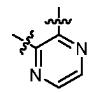,

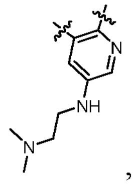, 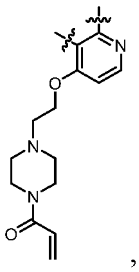, 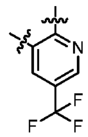, 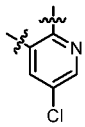 or 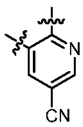.

**8.** The compound or a pharmaceutically acceptable salt, solvate, chelate, non-covalent complex, or prodrug thereof of any one of claims 1-3, wherein $R_1$ is hydrogen, $R_2$ is selected from the group consisting of $C_{5-6}$ cycloalkyl, substituted $C_{5-6}$ cycloalkyl, $C_{5-7}$ heterocyclyl, substituted $C_{5-7}$ heterocyclyl and phenyl, wherein the $C_{5-7}$ heterocyclyl optionally containing 1 or 2 heteroatoms selected from N and O.

**9.** The compound or a pharmaceutically acceptable salt, solvate, chelate, non-covalent complex, or prodrug thereof of any one of claims 1-3, wherein $R_2$ is substituted with 1 or 2 $R_{13}$ substituents, $R_{13}$ is selected from the group consisting of $C_{5-6}$ heterocyclyl, substituted $C_{5-6}$ heterocyclyl, $-NR_{14}R_{15}$ and $-CO-R_{16}$, $R_{14}$ is H, $R_{15}$ is $-CO-C_{2-4}$ alkenyl, $R_{16}$ is $C_{1-3}$ alkyl or substituted $C_{1-3}$ alkyl.

**10.** The compound or a pharmaceutically acceptable salt, solvate, chelate, non-covalent complex, or prodrug thereof of any one of claims 1-3, wherein $R_{13}$ is substituted with 0-1 $R_{18}$ substituent, $R_{18}$ is selected from the group consisting of $C_{1-6}$ alkyl, $C_{5-6}$ heterocyclyl and substituted $C_{5-6}$ heterocyclyl, wherein the $C_{5-6}$ heterocyclyl containing 1 or 2 heteroatoms selected from N and O.

**11.** The compound or a pharmaceutically acceptable salt, solvate, chelate, non-covalent complex, or prodrug thereof of any one of claims 1-3, wherein $R_2$ is

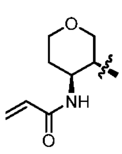, 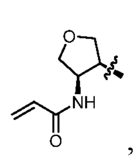,

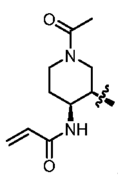, 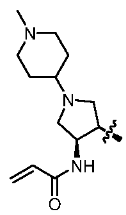 or 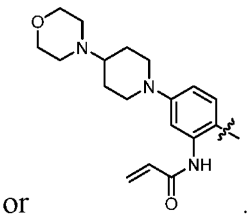.

**12.** The compound or a pharmaceutically acceptable salt, solvate, chelate, non-covalent complex, or prodrug thereof of any one of claims 1-3, wherein $R_5$, $R_6$, $R_7$ and $R_8$ are each independently selected from the group consisting of hydrogen, halogen, $C_{1-3}$ alkoxy and substituted $C_{1-3}$ alkoxy.

**13.** The compound or a pharmaceutically acceptable salt, solvate, chelate, non-covalent complex, or prodrug thereof of any one of claims 1-12, wherein $R_5$ and $R_8$ are selected from the following groups:

(i) both $R_5$ and $R_8$ are chlorine;
(ii) both $R_5$ and $R_8$ are hydrogen;
(iii) $R_5$ is hydrogen, $R_8$ is chlorine; or
(iv) $R_5$ is chlorine, $R_8$ is hydrogen.

**14.** The compound or a pharmaceutically acceptable salt, solvate, chelate, non-covalent complex, or prodrug thereof of any one of claims 1-13, wherein $R_6$ and $R_7$ are selected from the following groups:

(i) both $R_6$ and $R_7$ are methoxy;
(ii) $R_6$ is methoxy, $R_7$ is H; or
(iii) $R_6$ is H, $R_7$ is methoxy.

**15.** The compound or a pharmaceutically acceptable salt, solvate, chelate, non-covalent complex, or prodrug thereof of any one of claims 1-14, wherein m is 0 or 1.

**16.** The compound or a pharmaceutically acceptable salt, solvate, chelate, non-covalent complex, or prodrug thereof of any one of claims 1-15, wherein n is 2.

**17.** A compound or a pharmaceutically acceptable salt, wherein the compound is selected from:

(1) *N*-(2-((6-(2,6-dichloro-3,5-dimethoxyphenyl)-5,6-dihydropyrimido[5,4-*c*][1,8] naphthyridin-2-yl)amino)-5-(4-morpholinopiperidin-1-yl)phenyl)acrylamide;
(2) *N*-((3*S*,4*S*)-3-((6-(2,6-dichloro-3,5-dimethoxyphenyl)-5,6-dihydropyrimido[5,4-*c*][1,8] naphthyridin-2-yl)amino)tetrahydro-2*H*-pyran-4-yl)acrylamide;
(3) *N*-((3*S*,4*S*)-3-((6-(2,6-dichloro-3,5-dimethoxyphenyl)-5-oxo-5,6-dihydropyrimido [5,4-*c*][1,8]naphthyridin-2-yl)amino)tetrahydro-2*H*-pyran-4-yl)acrylamide;
(4) *N*-((3*S*,4*S*)-3-((6-(2,6-dichloro-3,5-dimethoxyphenyl)-5-methyl-5,6-dihydropyrimido [5,4-*c*][1,8]naphthyridin-2-yl)amino)tetrahydro-2*H*-pyran-4-yl)acrylamide;
(5) *N*-((3*S*,4*S*)-3-((6-(2,6-dichloro-3,5-dimethoxyphenyl)-5,6-dihydropyrimido [5,4-*e*][1,2,4]triazolo[4,3-*a*]pyrimidin-2-yl)amino)tetrahydro-2*H*-pyran-4-yl)acrylamide;
(6) *N*-((3*S*,4*S*)-3-((6-(2,6-dichloro-3,5-dimethoxyphenyl)-5,6-dihydropyrimido [5,4-*c*]quinolin-2-yl)amino)tetrahydro-2*H*-pyran-4-yl)acrylamide;
(7) *N*-((3*R*,4*S*)-4-((6-(2,6-dichloro-3,5-dimethoxyphenyl)-5,6-dihydropyrimido [5,4-*c*][1,8]naphthyridin-2-yl)amino)tetrahydrofuran-3-yl)acrylamide;
(8) *N*-((3*S*,4*S*)-3-((6-(2,6-dichloro-3,5-dimethoxyphenyl)-5,6-dihydropyrimido[5,4-*c*][1,5] naphthyridin-2-yl)amino)tetrahydro-2*H*-pyran-4-yl)acrylamide;
(9) *N*-((3*S*,4*S*)-3-((6-(2,6-dichloro-3,5-dimethoxyphenyl)-5,6-dihydropyrido[2,3-*d*:4,5-*d'*] dipyrimidin-2-yl)amino)tetrahydro-2*H*-pyran-4-yl)acrylamide;
(10) *N*-((3*S*,4*S*)-3-((6-(2,6-dichloro-3,5-dimethoxyphenyl)-5,6-dihydropyrazino[2',3':5,6] pyrido[4,3-*d*]pyrimidin-2-yl)amino)tetrahydro-2*H*-pyran-4-yl)acrylamide;
(11) *N*-((3*S*,4*S*)-3-((6-(2,6-dichloro-3,5-dimethoxyphenyl)-9-fluoro-5,6-dihydropyrimido [5,4-*c*][1,8]naphthyridin-2-yl)amino)tetrahydro-2*H*-pyran-4-yl)acrylamide;
(12) *N*-((3*S*,4*S*)-3-((6-(2,6-dichloro-3,5-dimethoxyphenyl)-10-methyl-5,6-dihydropyrimido[5,4-*c*][1,8]naphthyridin-2-yl)amino)tetrahydro-2*H*-pyran-4-yl)acrylamide;
(13) *N*-((3*S*,4*S*)-3-((6-(2,6-dichloro-3,5-dimethoxyphenyl)-9-methyl-5,6-dihydropyrimido [5,4-*c*][1,8]naphthyridin-2-yl)amino)tetrahydro-2*H*-pyran-4-yl)acrylamide;
(14) *N*-((3*S*,4*S*)-3-((6-(2-chloro-3,5-dimethoxyphenyl)-9-methyl-5,6-dihydropyrimido [5,4-*c*][1,8]naphthyridin-2-yl)amino)tetrahydro-2*H*-pyran-4-yl)acrylamide;
(15) *N*-((3*S*,4*S*)-3-((6-(2,6-dichloro-3,5-dimethoxyphenyl)-8-methyl-5,6-dihydropyrimido [5,4-*c*][1,8]naphthyridin-2-yl)amino)tetrahydro-2*H*-pyran-4-yl)acrylamide;
(16) *N*-((3*S*,4*S*)-3-((6-(2,6-dichloro-3,5-dimethoxyphenyl)-9-(trifluoromethyl)-5,6-dihydropyrimido[5,4-*c*][1,8]naphthyridin-2-yl)amino)tetrahydro-2*H*-pyran-4-yl)acrylamide;
(17) *N*-((3*S*,4*S*)-3-((6'-(2,6-dichloro-3,5-dimethoxyphenyl)-6'*H*-spiro[cyclopropane-1,5'-pyrimido[5,4-*c*][1,8]naphthyridin]-2'-yl)amino)tetrahydro-2*H*-pyran-4-yl)acrylamide;
(18) *N*-((3*S*,4*S*)-3-((6-(2,6-dichloro-3,5-dimethoxyphenyl)pyrimido[4,5-*f*][1,7] naphthyridin-2-yl)amino)tetrahydro-2*H*-pyran-4-yl)acrylamide;
(19) *N*-((3*S*,4*S*)-3-((6-(2,6-dichloro-3,5-dimethoxyphenyl)-5-methyl-5,6-dihydrothieno [3',4':5,6]pyrido[4,3-*d*]pyrimidin-2-yl)amino)tetrahydro-2*H*-pyran-4-yl)acrylamide;
(20) *N*-((3*S*,4*S*)-3-((6-(2,6-dichloro-3,5-dimethoxyphenyl)-6*H*-pyrido[3',2':4,5]pyrano [3,2-*d*]pyrimidin-2-yl)amino)tetrahydro-2*H*-pyran-4-yl)acrylamide;
(21) *N*-((3*S*,4*S*)-3-((6-(2-chloro-5-methoxyphenyl)-5,6-dihydropyrimido[5,4-*c*][1,8] naphthyridin-2-yl)amino)tetrahydro-2*H*-pyran-4-yl)acrylamide;
(22) *N*-((3*S*,4*S*)-3-((6-(2,6-dichloro-3,5-dimethoxyphenyl)-6,9,10,11-tetrahydro-5*H* -[1,4]oxazino[2,3-b]pyrimido[4,5-f][1,8]naphthyridin-2-yl)amino)tetrahydro-2*H*-pyran-4-yl )acrylamide;
(23) *N*-((3*R*,4*S*)-4-((10-(2-(4-acryloylpiperazin-1-yl)ethoxy)-6-(2,6-dichloro-3,5 -dimethoxyphenyl)-5,6-dihydropyrimido[5,4-*c*][1,8]naphthyridin-2-yl)amino)tetrahydrofur an-3-yl)acrylamide;
(24) *N*-((3*R*,4*S*)-1-acetyl-3-((6-(2,6-dichloro-3,5-dimethoxyphenyl)-5,6-dihydropyrimido [5,4-*c*][1,8]naphthyridin-2-yl)amino)piperidin-4-yl)acrylamide;
(25) *N*-((3*S*,4*S*)-3-((4-(2,6-dichloro-3,5-dimethoxyphenyl)-5-methyl-4,5-dihydrothiazolo [5',4':5,6]pyrido[4,3-d]pyrimidin-8-yl)amino)tetrahydro-2*H*-pyran-4-yl)acrylamide;
(26) *N*-((3*S*,4*S*)-3-((6-(2,6-dichloro-3,5-dimethoxyphenyl)-9-((2-(dimethylamino)ethyl) amino)-5,6-dihydropyrimido[5,4-*c*][1,8]naphthyridin-2-yl)amino)tetrahydro-2*H*-pyran-4-yl )acrylamide;
(27) *N*-((3*S*,4*R*)-4-((6-(2,6-dichloro-3,5-dimethoxyphenyl)-5,6-dihydropyrimido [5,4-*c*][1,8]naphthyridin-2-yl)amino)-1-(1-methylpiperidin-4-yl)pyrrolidin-3-yl)acrylamide;
(28) *N*-((3*S*,4*S*)-3-((9-chloro-6-(2,6-dichloro-3,5-dimethoxyphenyl)-5,6-dihydropyrimido[5,4-*c*][1,8]naphthyridin-2-yl)amino)tetrahydro-2*H*-pyran-4-yl)acrylamide;
(29) *N*-((3*S*,4*S*)-3-((9-cyano-6-(2,6-dichloro-3,5-dimethoxyphenyl)-5,6-dihydropyrimido [5,4-*c*][1,8]naphthyridin-2-yl)amino)tetrahydro-2*H*-pyran-4-yl)acrylamide;
(30) *N*-((3*S*,4*S*)-3-((6-(2-chloro-5-methoxyphenyl)-9-methyl-5,6-dihydropyrimido [5,4-*c*][1,8]naphthyridin-2-yl)amino)tetrahydro-2*H*-pyran-4-yl)acrylamide;
(31) *N*-((3*S*,4*S*)-3-((6-(2-chloro-3-methoxyphenyl)-9-methyl-5,6-dihydropyrimido [5,4-*c*][1,8]naphthyridin-2-yl)amino)tetrahydro-2*H*-pyran-4-yl)acrylamide;
(32) *N*-((3*S*,4*S*)-3-((6-(2,6-dichloro-3-methoxyphenyl)-9-methyl-5,6-dihydropyrimido [5,4-*c*][1,8]naphthyridin-2-yl)amino)tetrahydro-2*H*-pyran-4-yl)acrylamide;
(33) *N*-((3*S*,4*S*)-3-((6-(2,6-dichloro-3,5-dimethoxyphenyl)-[1,2,4]triazolo [4',3':1,6]pyrido[2,3-d]pyrimidin-2-yl)amino)tetrahydro-2*H*-pyran-4-yl)acrylamide; or
(34) *N*-(3-((9-chloro-6-(2,6-dichloro-3,5-dimethoxyphenyl)-5-oxo-5,6-dihydropyrimido [5,4-*c*][1,8]naphthyridin-2-yl)amino)tetrahydro-2*H*-pyran-4-yl)acrylamide.

**18.** A pharmaceutical composition comprsing a therapeutically effect amount of compound or a pharmaceutically acceptable salt, solvate, chelate, non-covalent complex, or prodrug thereof according to any one of claims 1-17 and at least one pharmaceutically acceptable excipient.

**19.** A pharmaceutical composition of claim 18, wherein a mass ratio of the said compound and the pharmaceutically acceptable excipient is 0.0001:1-10.

**20.** Use of the compound or a pharmaceutically acceptable salt, solvate, chelate, non-covalent complex, or prodrug thereof according to any one of claims 1-17 or the pharmaceutical composition according to claim 18 or 19 in the manufacture of a medicament.

**21.** The use of claim 20, wherein the medicament is used in the treatment, prevention or delay of the occurrence or progression of cancer or cancer metastasis.

**22.** Use of the compound or a pharmaceutically acceptable salt, solvate, chelate, non-covalent complex, or prodrug thereof according to any one of claims 1-17 or the pharmaceutical composition according to claim 18 or 19 in the manufacture of a medicament for use in the treatment of disease mediated by FGFR4.

**23.** The use of claim 22, wherein the disease is cancer.

**24.** The use of claim 22 or 23, wherein the cancer is selected from the group consisting of breast cancer, multiple myeloma, bladder cancer, endometrial cancer, gastric cancer, cervical cancer, rhabdomyosarcoma, non-small cell lung cancer, small cell lung cancer, pleomorphic lung cancer, ovarian cancer, esophageal cancer, melanoma, colorectal cancer , Hepatocellular carcinoma, head and neck tumors, hepatobiliary cell carcinoma, myelodysplastic

syndrome, malignant glioma, prostate cancer, thyroid cancer, Schwann cell tumor, lung squamous cell carcinoma, lichenoid keratosis, Synovial sarcoma, skin cancer, pancreatic cancer, testicular cancer or liposarcoma.

**25.** The use of claim 20, wherein the medicament is used as FGFR4 inhibitor.

**26.** A method of treating or preventing disease mediated FGFR4, comprising administering a therapeutically effective amount of the compound or a pharmaceutically acceptable salt, solvate, chelate, non-covalent complex, or prodrug thereof according to any one of claims 1-17 or the pharmaceutical composition according to claims 18-19 to a subject.

**27.** The method of claim 26, wherein the disease mediated by FGFR4 is cancer.

**28.** The method of claim 27, wherein the cancer is selected from the group consisting of breast cancer, multiple myeloma, bladder cancer, endometrial cancer, gastric cancer, cervical cancer, rhabdomyosarcoma, non-small cell lung cancer, small cell lung cancer, pleomorphic lung cancer, ovarian cancer, esophageal cancer, melanoma, colorectal cancer, hepatocellular carcinoma, head and neck tumors, hepatobiliary cell carcinoma, myelodysplastic syndrome, malignant glioma, prostate cancer, thyroid cancer, Schwann cell tumor, lung squamous cell carcinoma, lichenoid keratosis, synovial sarcoma, skin cancer, pancreatic cancer, testicular cancer or liposarcoma.

**29.** A method of treating cancer comprising administering a therapeutical effective amount of a compound or a pharmaceutically acceptable salt, solvate, chelate, non-covalent complex, or prodrug thereof of any one of claims 1-17 or a pharmaceutical composition of claim 18 or 19 to a subject, wherein the cancer is breast cancer, multiple myeloma, bladder cancer, endometrial cancer, gastric cancer, cervical cancer, rhabdomyosarcoma, non-small cell lung cancer, small cell lung cancer, pleomorphic lung cancer, ovarian cancer, esophageal cancer, melanoma, colorectal cancer, hepatocellular carcinoma, head and neck tumors, hepatobiliary cell carcinoma, myelodysplastic syndrome, malignant glioma, prostate cancer, thyroid cancer, Schwann cell tumor, lung squamous cell carcinoma, lichenoid keratosis, synovial sarcoma, skin cancer, pancreatic cancer, testicular cancer or liposarcoma.

**30.** The method of any one of claims 26-29, wherein the subject is human.

**INTERNATIONAL SEARCH REPORT**

International application No.
**PCT/CN2019/108298**

**A. CLASSIFICATION OF SUBJECT MATTER**

C07D 487/14(2006.01)i; C07D 471/14(2006.01)i; C07D 519/00(2006.01)i; A61K 31/4375(2006.01)i; A61K 31/437(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D;A61K;A61P

Documentation searched other than minimum documentation to the extent that such documents are included in the fields searched

Electronic data base consulted during the international search (name of data base and, where practicable, search terms used)

WPI; EPODOC; STN; CNABS; CNTXT; USTXT; EPTXT; WOTXT; 贝达药业, 高金恒, 孙中心, 张赟, 徐晓峰, 刘湘永, 王家炳, 丁列明, 成纤维细胞, 嘧啶, 丙烯酰胺, 萘啶, 吡喃, 吡咯, FGFR4, pyrimid+, acrylamide, naphthyridi+, pyran, pyrrole, fibroblast, apoptosis; 1395027-49-2, 1395032-38-8, 式 (I) 及具体化合物的结构式检索, structural search according to formula I and specific compounds

**C. DOCUMENTS CONSIDERED TO BE RELEVANT**

| Category* | Citation of document, with indication, where appropriate, of the relevant passages | Relevant to claim No. |
|---|---|---|
| X | WO 2018004258 A1 (HANMI PHARMACEUTICAL CO., LTD.) 04 January 2018 (2018-01-04)<br>the abstract, and description, paragraphs [0197], [0246] and [0342] | 1-30 |
| X | CN 104271576 A (ABBVIE INC.) 07 January 2015 (2015-01-07)<br>the abstract, and description, paragraphs [0360], [0363] and [0366] | 1, 4-7, 9, 10, 12, 13, 15, 18, 20, 21 |
| X | WO 2018153373 A1 (BETTA PHARMACEUTICALS CO., LTD.) 30 August 2018 (2018-08-30)<br>the abstract, and description, pages 18, 19, 36-43, 46, 47 and 51 | 1-30 |
| A | CN 103703005 A (ABBVIE INC.) 02 April 2014 (2014-04-02)<br>the abstract, and description, paragraph [0280] | 1-30 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **03 December 2019** | **03 January 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No. **PCT/CN2019/108298**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **26-30**
because they relate to subject matter not required to be searched by this Authority, namely:

[1] The subject matter of claims 26-30 is "a method for treating and/or preventing a disease mediated by FGFR4" or "a method for treating cancer", which relates to a living human or animal body, and is a prevention or treatment method for disease. This report is made on the basis of pharmaceutical application.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2019/108298**

| Patent document cited in search report | Publication date (day/month/year) | Patent family member(s) | Publication date (day/month/year) |
|---|---|---|---|
| WO 2018004258 A1 | 04 January 2018 | None | |
| CN 104271576 A | 07 January 2015 | ES 2606640 T3 | 24 March 2017 |
| | | EP 2817308 B1 | 07 September 2016 |
| | | US 9718821 B2 | 01 August 2017 |
| | | CA 2864142 A1 | 29 August 2013 |
| | | US 2013225589 A1 | 29 August 2013 |
| | | EP 2817308 A1 | 31 December 2014 |
| | | MX 2014010176 A | 10 November 2014 |
| | | WO 2013126656 A1 | 29 August 2013 |
| | | JP 2015511245 A | 16 April 2015 |
| WO 2018153373 A1 | 30 August 2018 | TW 201831483 A | 01 September 2018 |
| | | SG 11201907909 T A | 27 September 2019 |
| | | AU 2018226315 A1 | 03 October 2019 |
| | | CA 3054455 A1 | 30 August 2018 |
| | | CN 110382499 A | 25 October 2019 |
| CN 103703005 A | 02 April 2014 | MX 340402 B | 07 July 2016 |
| | | JP 5886326 B2 | 16 March 2016 |
| | | WO 2012161812 A1 | 29 November 2012 |
| | | CA 2827648 A1 | 29 November 2012 |
| | | UY 33925 A | 28 September 2012 |
| | | TW 201247675 A | 01 December 2012 |
| | | TW I532742 B | 11 May 2016 |
| | | EP 2681221 B1 | 18 May 2016 |
| | | ES 2587514 T3 | 25 October 2016 |
| | | MX 2013009873 A | 12 March 2014 |
| | | US 8710065 B2 | 29 April 2014 |
| | | AR 085502 A1 | 09 October 2013 |
| | | JP 2014506929 A | 20 March 2014 |
| | | EP 2681221 A1 | 08 January 2014 |
| | | US 2012220572 A1 | 30 August 2012 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015061572 A


### Non-patent literature cited in the description


- **JOUANNEAU J et al.** *Oncogene,* 1999, vol. 18, 327-333
- **KOSTRZEWA M.** *Mammalian genome,* 1998, vol. 9 (2), 131-135
- **ESWARAKUMAR V P et al.** *Cytokine Growth Factor Rev,* 2005, vol. 16 (2), 139-149
- **SCHLESSINGER J et al.** *Mol Cell,* 2000, vol. 6, 743-750
- **DAILEY L et al.** *Cytokine Growth Factor Rev,* 2005, vol. 16, 233-247
- Design of Prodrugs. Elsevier, 1985